(19) Europäisches Patentamt
European Patent Office
Office européen des brevets

(11) **EP 4 289 966 A1**

(12) **EUROPEAN PATENT APPLICATION**
published in accordance with Art. 153(4) EPC

(43) Date of publication:
**13.12.2023  Bulletin 2023/50**

(21) Application number: **21924592.5**

(22) Date of filing: **03.02.2021**

(51) International Patent Classification (IPC):
**C12Q 1/6869** (2018.01)

(52) Cooperative Patent Classification (CPC):
**C12Q 1/6869; C12Q 1/68; G16B 30/10**

(86) International application number:
**PCT/JP2021/003874**

(87) International publication number:
**WO 2022/168195 (11.08.2022 Gazette 2022/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Tohoku University**
**Sendai-shi, Miyagi 980-8577 (JP)**

(72) Inventors:
• **MATSUO Ayumi**
**Sendai-shi, Miyagi 980-8577 (JP)**
• **SUYAMA Yoshihisa**
**Sendai-shi, Miyagi 980-8577 (JP)**
• **SATO Mitsuhiko**
**Sendai-shi, Miyagi 980-8577 (JP)**
• **HIROTA Shun**
**Sendai-shi, Miyagi 980-8577 (JP)**

(74) Representative: **Plasseraud IP**
**66, rue de la Chaussée d'Antin**
**75440 Paris Cedex 09 (FR)**

Remarks:
The complete document including Reference
Table(s) and the Sequence Listing(s) can be
downloaded from the EPO website

(54) **GENETIC INFORMATION ANALYSIS SYSTEM AND GENETIC INFORMATION ANALYSIS METHOD**

(57)  An aspect of the present invention is a genetic information analysis system including: a comparison-side information acquisition unit configured to acquire comparison target sequence information indicating a comparison target sequence which is a base sequence of genetic information on a comparison target; and an accuracy acquisition unit configured to acquire an accuracy that a type of a plant as the comparison target is the same as a type of a plant as an analysis target, based on the comparison target sequence information and reference sequence information indicating a reference sequence selected from base sequences common to two or more independently acquired base sequences of the genetic information on the analysis target.

**Description**

Technical Field

The present invention relates to a genetic information analysis system and a genetic information analysis method.

Background Art

In agricultural, forestry and fishery products, development of a technique for identifying differences between species, production areas, individuals, and varieties accurately and quickly at a low cost has become an urgent issue for preventing variety falsification (in particular, infringement of plant breeder's rights, falsification of production area, quality falsification, and the like). DNA identification methods of species, production areas, individuals, and varieties that have been used in the related art are mainly classified into the following two types.

One is a method that does not require prior acquisition of DNA information. Examples of such a method include: (1) a method of detecting a difference in length of DNA fragments by restriction enzyme treatment (RFLP or the like); (2) a method of comparing the presence or absence of an amplified fragment by PCR (RAPD, ISSR, or the like); and (3) a method of detecting the presence or absence of a DNA fragment by gel electrophoresis or the like by a combination of the above two methods (AFLP or the like). Without requiring a prior information acquisition operation, these methods have an advantage of high versatility, but are not used much at present due to poor reproducibility and identification ability.

In recent years, a method of performing SNP(s) genotyping by genome analysis using a next-generation sequencer has been proposed (for example, NPL 1), and is expected as a method for quickly and accurately identifying the difference between varieties.

The other is a method of acquiring DNA information on an analyte target variety in advance, searching for a region effective for identification in a genomic DNA of the target variety, and performing identification by using a PCR primer created for detecting (amplifying) only the region (for example, PTL 1, NPLs 1 and 2) . That is, the method is a method of performing difference identification using DNA information on only a marker (DNA marker) region for identification, such as microsatellite analysis. This method generally uses DNA mutation information on a region that has been confirmed to be detectable in advance is used, and thus has a high reproducibility of detection data. On the other hand, this method has many difficulties. For example, the DNA marker has no versatility, that is, a marker needs to be developed for each classification group. Moreover, a large amount of time and cost is required for a screening operation for selecting an effective DNA marker. Furthermore, identification information may be insufficient if the developed DNA marker has low mutability.

Citation list

Patent Literature

PTL 1: Japanese Patent No. 6220332

Non-Patent Literature

NPL 1: Suyama Y, Matsuki Y (2015) MIG-seq: an effective PCR-based method for genome-wide single-nucleotide polymorphism genotyping using the next-generation sequencing platform. Scientific Reports 5: 16963.

Summary of Invention

Technical Problem

In recent years, attention has been paid to a technique for identifying a difference between varieties by SNP genotyping using a next-generation sequencer. However, base sequence information obtained from the next-generation sequencer has a disadvantage of containing errors at a high rate (error ratio: 0.1% to 16%; Travis C Glenn, Mol Ecol Resour. 2011 Sep; 11(5): 759-69.). Since an enormous number of sequences can be obtained from an extremely large number of regions in a genome, it is not rare that the same or substantially the same sequence that is originally present in a plurality of different regions in the genome is obtained. Therefore, except for organisms for which entire genome information has already been obtained, it is difficult to perform data analysis when comparing sequence data between different samples, especially in the case of genome duplication (a phenomenon in which a part of the genome is duplicated and almost the same sequence exists in a plurality of locations) and the case of a higher-order multiple (a configuration in which the entire genome is duplicated a plurality of times), or the like.

Furthermore, in a method of acquiring base sequence data on a plurality of genome-wide regions by the next-generation sequencer, sequence data on all regions cannot always be obtained, and data loss generally occurs. Therefore, it is not possible to simply evaluate a genomic similarity between the samples depending on the presence or absence of the base sequence data.

A base sequence obtained by a sequencer, without being limited to the next-generation sequencer, contains not a few errors. Therefore, in order to evaluate the similarity between the samples based on the base sequence data obtained by the sequencer, it is necessary to select highly accurate base sequence data that can withstand the evaluation of the similarity.

Accordingly, an object of the invention is to provide a genetic information analysis system and a genetic information analysis method that can evaluate similarity between a plurality of biological samples. Another object of the invention is to provide a reference sequence information acquisition device and a reference sequence information acquisition method that can provide highly accurate base sequence data with fewer errors. Still another object of the invention is to provide a specific sequence information acquisition system and a specific sequence information acquisition method for acquiring a base sequence specific to a reference sequence acquired by the reference sequence information acquisition device or the reference sequence information acquisition method.

Solution to Problem

According to an aspect of the invention, a genetic information analysis system includes: a comparison-side information acquisition unit configured to acquire comparison target sequence information indicating a comparison target sequence which is a base sequence of genetic information on a comparison target; and an accuracy acquisition unit configured to acquire an accuracy that the genetic information on the comparison target is the same as genetic information on an analysis target, based on the comparison target sequence information and reference sequence information indicating a reference sequence selected from base sequences common to two or more independently acquired base sequences of the genetic information on the analysis target.

According to an aspect of the invention, in the gene information analysis system, the reference sequence information is acquired based on at least first base sequence information indicating one of the two or more independently acquired base sequences of the genetic information on the analysis target, and second base sequence information indicating another base sequence of the two or more base sequences.

According to an aspect of the invention, in the gene information analysis system, a sequence information amount of the base sequence of the comparison target is larger than a sequence information amount of the reference sequence.

According to an aspect of the invention, a genetic information analysis method includes: a step of acquiring comparison target sequence information indicating a comparison target sequence which is a base sequence of genetic information on a comparison target; and a step of acquiring an accuracy that the genetic information on the comparison target is the same as genetic information on an analysis target, based on the comparison target sequence information and reference sequence information indicating a reference sequence selected from base sequences common to two or more independently acquired base sequences of the genetic information on the analysis target.

According to an aspect of the invention, in the gene information analysis method, the reference sequence information is acquired based on at least first base sequence information indicating one of the two or more independently acquired base sequences of the genetic information on the analysis target, and second base sequence information indicating another base sequence of the two or more base sequences.

According to an aspect of the invention, in the gene information analysis method, a sequence information amount of the base sequence of the comparison target is larger than a sequence information amount of the reference sequence.

According to an aspect of the invention, a reference sequence information acquisition device includes a reference sequence information acquisition unit configured to acquire reference sequence information indicating a reference sequence selected from base sequences common to two or more independently acquired base sequences of genetic information on an analysis target.

According to an aspect of the invention, in the reference sequence information acquisition device, a reference sequence information acquisition method includes a step of acquiring reference sequence information indicating a reference sequence selected from base sequences common to two or more independently acquired base sequences of genetic information on an analysis target.

According to an aspect of the invention, a specific sequence information acquisition system includes: a reference sequence information acquisition unit configured to acquire reference sequence information indicating a reference sequence selected from base sequences common to two or more independently acquired base sequences of genetic information on an analysis target; and a specific sequence information acquisition unit configured to acquire, from the reference sequence information, specific sequence information indicating a base sequence that is specifically present in the reference sequence.

According to an aspect of the invention, a specific sequence information acquisition method includes: a step of acquiring reference sequence information indicating a reference sequence selected from base sequences common to two or more independently acquired base sequences of genetic information on an analysis target; and a step of acquiring, from the reference sequence information, specific sequence information indicating a base sequence that is specifically present in the reference sequence.

According to an aspect of the invention, a method for producing a detection nucleic acid includes: a step of acquiring specific sequence information by the reference sequence information acquisition method; and a step of producing a detection nucleic acid of the analysis target based on the specific sequence information.

Advantageous Effect

The invention provides a genetic information analysis system and a genetic information analysis method that can evaluate similarity between a plurality of biological samples. In addition, the invention provides a reference sequence information acquisition device and a reference sequence information acquisition method that can provide highly accurate base sequence data with fewer errors. Further, the invention provides a specific sequence information acquisition system and a specific sequence information acquisition method for acquiring a base sequence specific to a reference sequence acquired by the reference sequence information acquisition device or the reference sequence information acquisition method. Furthermore, the invention provides a method for producing a detection nucleic acid that enables highly accurate detection of genetic information on a target based on the specific sequence.

Brief Description of Drawings

[FIG. 1] FIG. 1 is a diagram showing an example of a configuration of a genetic information analysis system 100 according to an embodiment.

[FIG. 2] FIG. 2 is a diagram showing an example of a method for acquiring a reference sequence according to the embodiment.

[FIG. 3] FIG. 3 is a diagram showing an example of a hardware configuration of a reference sequence information acquisition device 1 according to the embodiment.

[FIG. 4] FIG. 4 is a diagram showing an example of a hardware configuration of an evaluation device 2 according to the embodiment.

[FIG. 5] FIG. 5 is a diagram showing an example of a functional configuration of a control unit 11 according to the embodiment.

[FIG. 6] FIG. 6 is a diagram showing an example of a functional configuration of a control unit 21 according to the embodiment.

[FIG. 7] FIG. 7 is a flowchart showing an example of a flow of processing executed by the reference sequence information acquisition device 1 according to the embodiment.

[FIG. 8] FIG. 8 is a flowchart showing an example of a flow of processing executed by the evaluation device 2 according to the embodiment.

[FIG. 9] FIG. 9 is a diagram showing an example of a configuration of a specific sequence information acquisition system 200 according to the embodiment.

[FIG. 10] FIG. 10 is a diagram showing an example of a hardware configuration of a specific sequence information acquisition device 3 according to the embodiment.

[FIG. 11] FIG. 11 is a diagram showing an example of a functional configuration of a control unit 31 according to the embodiment.

[FIG. 12] FIG. 12 is a flowchart showing an example of a flow of processing executed by the specific sequence information acquisition device 3 according to the embodiment.

[FIG. 13] FIG. 13 is a diagram showing an overview of MIG-seq (modified based on Yoshihisa Suyama (2019), Use of MIG-seq in forest genetic and breeding studies. Forest Genetics and Breeding 8(2): 85-89). SSR: simple sequence repeat, ISSR: inter-simple sequence repeat, multiplex PCR: PCR that amplifies two or more genes by the same reaction.

[FIG. 14] FIG. 14 is a diagram showing parent-child correlations of shiitake mushroom varieties used in examples.

[FIG. 15] FIG. 15 is a diagram showing a result obtained by performing similarity evaluation using reference sequence information acquired from C-1 in Example 1.

[FIG. 16] FIG. 16 is a diagram showing a result obtained by performing similarity evaluation using reference sequence information acquired from C-2 in Example 1.

[FIG. 17] FIG. 17 is a diagram showing a result obtained by performing similarity evaluation using reference sequence information acquired from a variety D in Example 1.

[FIG. 18] FIG. 18 is a diagram showing a result obtained by performing PCR using a genomic DNA of each one among varieties A to E as a template using a variety D specific primer obtained in Example 2.

Description of Embodiments

[Gene information analysis system]

For simplification of the description, a case will be described below as an example in which an analysis target sample is a biological sample. However, the analysis target is not particularly limited as long as genetic information is contained. The genetic information is usually base sequence information such as DNA and RNA. More specifically, examples of the analysis target include viruses, bacteria, archaea, fungi, algae, protozoa, plants, and animals. Examples of the genetic information include genomic DNA, genomic RNA of the virus or organism, or mRNA which is a transcription product thereof. The "biological sample" in the following description is a sample containing genetic information (genomic DNA, mRNA, or the like) derived from the organism as the analysis target, and may be in any form as long as the genetic information remains. Examples of the biological sample include, but are not limited to, living organisms, dried bodies, dried powders, processed biological products, and cultured cells.

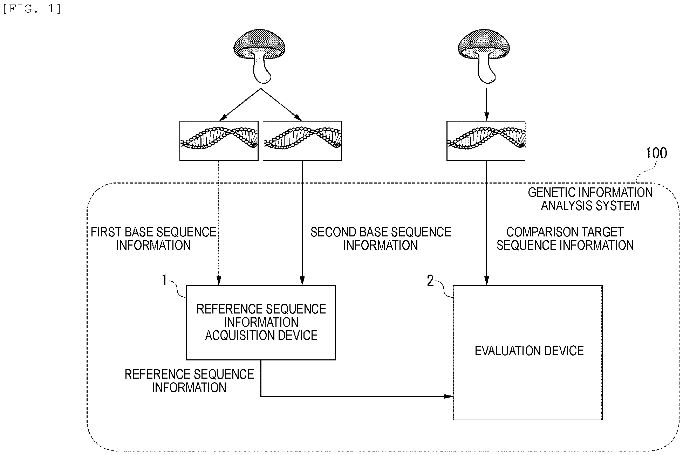

FIG. 1 is a diagram showing an example of a configuration of a genetic information analysis system 100 according to an embodiment. The genetic information analysis system 100 acquires a matching accuracy. The matching accuracy is an accuracy that a type of an organism as an analysis target is the same as a type of an organism as a comparison target. That is, the matching accuracy is information indicating an accuracy that an evaluation result of a magnitude of a degree of similarity of the organism as the analysis target with respect to the organism as the comparison target is correct. The matching accuracy indicates an accuracy that genetic information on the organism as the comparison target and genetic information on the organism as the analysis target are the same.

The genetic information analysis system 100 includes a reference sequence information acquisition device 1 and an evaluation device 2.

The reference sequence information acquisition device 1 executes reference sequence information acquisition. The reference sequence information acquisition is processing for acquiring information indicating a reference sequence (hereinafter, referred to as "reference sequence information") based on at least two pieces of analysis sequence information including first base sequence information and second base sequence information.

The analysis sequence information is a base sequence of nucleic acids, which is genetic information obtained from a biological sample of an analysis target. The nucleic acids, which are the genetic information, may be DNA or RNA, and is preferably genomic DNA. Both the first base sequence information and the second base sequence information are examples of analysis sequence information.

A method for acquiring analysis sequence information from a biological sample of an analysis target is not particularly limited, and a publicly known method can be used. For example, the analysis sequence information can be acquired by extracting nucleic acids from the biological sample, preparing a sequence analysis sample by a publicly known nucleic acid amplification method (PCR, isothermal amplification, or the like), and then performing sequence analysis by a sequencer. A nucleic acid region from which a base sequence is acquired as the analysis sequence information is not particularly limited, and a base sequence of any region can be acquired. For example, the nucleic acid region may be a genomic region where the existence of genetic polymorphism is known, or the whole genome. The genetic polymorphism may be any one of substitution, insertion, deletion, and the number of repeats of bases, and may be a combination thereof. The genetic polymorphism may be a single nucleotide polymorphism (SNP). As an example, the sequence analysis sample may include a DNA fragment amplified by multiplex PCR (PCR in which two or more base sequences are amplified by the same reaction) using a primer designed from a plurality of simple repeat sequences using, as a template, a genomic DNA of the analysis target.

The sequencer to be used for the sequence analysis may be a sequencer based on Sanger method, or may be a next-generation sequencer or a further-next-generation sequencer, and is preferably a next-generation sequencer. The next-generation sequencer is a term used in contrast to a "first-generation sequencer" which is a sequencer by capillary electrophoresis using Sanger method, and is a sequencer that determines a base sequence by processing a large number of DNA fragments (several tens of millions to several billions) in parallel. Examples of a sequencing technique used in the next-generation sequencer include, but are not limited to, ion semiconductor sequencing, pyrosequencing, sequencing by synthesis using a reversible dye terminator, and sequencing by ligation. Examples of a sequencing technique used in the further-next-generation sequencer include, but are not limited to, nanopore sequencing (Nat. Bioltechnol. 26, 1146-1153 (2008); SCIENCE, 303, 1189-1192 (2004); Surface Science 432, L611-L616 (1999); Nano Lett., 279-285 (2011); Scientific Reports, 2, 501 (2012)).

The analysis sequence information is acquired based on two or more base sequences independently acquired from the same biological sample. The first base sequence information indicates a first base sequence. The first base sequence is one of the two or more independently acquired base sequences of genetic information on the analysis target. The second base sequence information indicates a second base sequence. The second base sequence is another one of the two or more independently acquired base sequences of the analysis target. The term "independently acquired" means being subjected to sequence analysis as independent samples in the sequence analysis by the sequencer. Examples of a method for independently sequencing include a method of separately performing sequence analysis in a sequencer, a method of adding different labeled sequences to perform sequence analysis by a next-generation sequencer, and distributing the acquired base sequence based on the labeled sequences. The first base sequence and the second base sequence can be acquired as follows, for example.

a) A nucleic acid sample is prepared by extracting nucleic acids from a biological sample, a sequence analysis sample is prepared and then divided into two or more, and separately subjected to sequence analysis to acquire a base sequence for each sequence analysis. One of the acquired base sequences is referred to as the first base sequence, and another one is referred to as the second base sequence. After the preparation of the nucleic acid sample, the nucleic acid sample may be divided into two or more and separately subjected to the preparation of the sequence analysis sample and the sequence analysis. Alternatively, the biological sample may be divided into two or more and separately subjected to the preparation of the nucleic acid sample, the preparation of the sequence analysis sample, and the sequence analysis.

b) A nucleic acid sample is prepared by extracting nucleic acids from a biological sample. The nucleic acid sample is divided into two or more, and added with different labeled sequences to prepare sequence analysis samples. The sequence analysis samples are collectively subjected to the sequence analysis using the next-generation sequencer. The acquired base sequences are divided based on the labeled sequences, and a base sequence is acquired for each labeled sequence. One of the acquired base sequences is referred to as the first base sequence, and another one is referred to as the second base sequence. The biological sample may be divided into two or more, and the preparation of the nucleic acid sample and the preparation of the sequence analysis sample may be performed.

In either one of the above a) and b), all operations performed from the extraction of the nucleic acids to the acquisition of the base sequences are preferably performed under the same condition in the acquisition of the first base sequence and the second base sequence.

The first base sequence and the second base sequence are preferably acquired by MIG-seq (Multiplexed ISSR Genotyping by sequencing; Suyama Y, Matsuki Y (2015) Scientific Reports 5: 16963.). Specific examples thereof include methods described in Examples.

When the first base sequence and the second base sequence are base sequences acquired by a next-generation sequencer, base sequences whose number of reads (the number of times that the same base sequence is read) is equal to or larger than a predetermined reference value may be selected as the first base sequence and the second base sequence. It is considered that a larger number of reads indicates a lower risk of containing sequencing errors and a higher reliability of the base sequence. It can be said a larger number of reads indicates a base sequence more representative for the genetic information on the biological sample of the analysis target. Examples of a reference value of the number of reads include 5 times or more, 7 times or more, or 10 times or more. The selection of the base sequence based on the number of reads may be performed at the time of selecting a reference sequence to be described later.

Hereinafter, for the sake of simplicity, the genetic information analysis system 100 will be described while being exemplified by a case in which the reference sequence information is acquired based on the first base sequence information and the second base sequence information.

The reference sequence is a base sequence selected from a base sequence common to the first base sequence and the second base sequence.

More specifically, the reference sequence information acquisition is, for example, processing for comparing the first base sequence information with the second base sequence information to detect a matching base sequence, and acquiring the matching base sequence as the reference sequence information. The matching base sequence is a reference sequence.

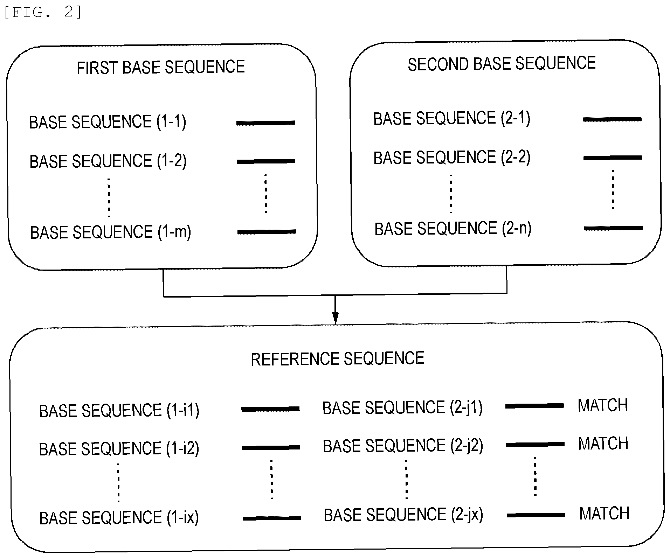

Each of the first base sequence and the second base sequence may be a set of base sequences including a plurality of base sequences. For example, the first base sequence may be a set of m base sequences including a base sequence (1-1) to a base sequence (1-m). The second base sequence may be a set of n base sequences including a base sequence (2-1) to a base sequence (2-n). The reference sequence may be a set of base sequences obtained by comparing the base sequence (1-1) to the base sequence (1-m) with the base sequence (2-1) to the base sequence (2-n) and selecting completely matched base sequences (see FIG. 2). A length of each base sequence including the first base sequence, the second base sequence, and the reference sequence is not particularly limited, and examples thereof include 100 to 500 bases, 100 to 400 bases, 100 to 300 bases, and 100 to 200 bases.

When the selection based on the number of reads is not performed at the time of acquisition of the first base sequence and the second base sequence, the selection based on the number of reads described above may be performed after the common sequences of the first base sequence and the second base sequence are detected.

The evaluation device 2 executes evaluation. The evaluation is processing for acquiring the matching accuracy based on the reference sequence information and information (hereinafter, referred to as "comparison target sequence information") indicating the base sequence of the organism as the comparison target (hereinafter, referred to as "comparison target sequence"). Since the matching accuracy is information indicating an accuracy that the evaluation result of the magnitude of the degree of similarity is correct, the evaluation is an example of processing for evaluating the similarity.

The comparison target sequence is a base sequence of genetic information on an organism whose matching accuracy with the biological sample, from which the reference sequence is obtained, is desired to be obtained. For example, in a case of performing variety determination of a product attached with a specific plant variety name, the reference sequence may be acquired from the product, and as the comparison target sequence, a base sequence acquired from a plant individual that is known to be the plant variety may be used. Alternatively, the comparison target sequence may be a base sequence published as the base sequence of the plant variety. The public information on the base sequence can be obtained from, for example, a gene database (GenBank, DDBJ, or the like) or a document.

A sequence information amount of the comparison target sequence is preferably equal to or larger than a sequence information amount of the first base sequence and the second base sequence. Accordingly, the sequence information on the first base sequence and the second base sequence can be used without waste, and a higher matching accuracy can be acquired. For example, a base sequence of a nucleic acid region including nucleic acid regions (for example, a genomic DNA region) of the first base sequence and the second base sequence can be used as the comparison target sequence. The comparison target sequence can be acquired as follows, for example.

A) Nucleic acids are extracted from the biological sample of the comparison target, and a base sequence is acquired under the same conditions (the same primers, the same amplification conditions, and the same sequence analysis conditions) as those of the first base sequence and the second base sequence.

B) Nucleic acids are extracted from the biological sample of the comparison target, and are subjected to nucleic acid amplification by using the same primers as those of the first base sequence and the second base sequence at an annealing temperature lower than the annealing temperature of the first base sequence and the second base sequence to prepare a sequence analysis sample. The sequence analysis is performed under the same sequence analysis conditions as those of the first base sequence and the second base sequence to acquire a base sequence.

C) Nucleic acids are extracted from the biological sample of the comparison target, and subjected to nucleic acid amplification under the same conditions (the same primers, the same amplification conditions) as those of the first base sequence and the second base sequence to prepare a sequence analysis sample. The sequence analysis is performed under a sequence analysis condition gentler than that of the first base sequence and the second base sequence to acquire a base sequence. For example, in the comparison target sequence, the selection of the sequence read by the sequencer (for example, the next-generation sequencer) is not performed, or the degree of selection is lower than that of the first base sequence and the second base sequence.

D) When a base sequence of a genomic region including the first base sequence and the second base sequence is publicly known for an organism as a comparison target, the known base sequence is acquired from a gene database, a document, or the like.

E) The whole genome data on the organism as the comparison target is used as the comparison target sequence. The whole genome data may be newly acquired from the biological sample of the comparison target, or may be acquired from a gene database, a document, or the like.

The comparison target sequence is preferably acquired by MIG-seq (Multiplexed ISSR Genotyping by sequencing; Suyama Y, Matsuki Y (2015) Scientific Reports 5: 16963.). Specific examples thereof include methods described in Examples.

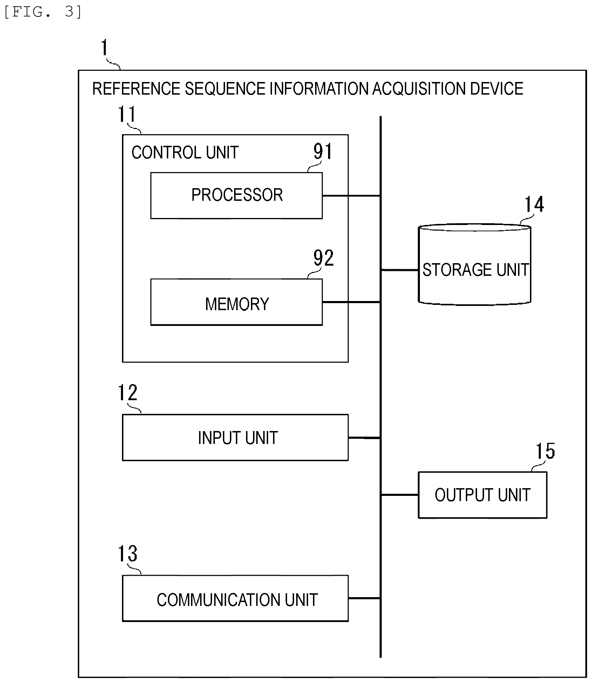

FIG. 3 is a diagram showing an example of a hardware configuration of a reference sequence information acquisition device 1 according to the embodiment. The reference sequence information acquisition device 1 includes a control unit 11 including a processor 91 such as a CPU and a memory 92 which are connected to a bus, and executes a program. The reference sequence information acquisition device 1 functions as a device including a control unit 11, an input unit 12, a communication unit 13, a storage unit 14, and an output unit 15 by executing a program.

More specifically, the processor 91 reads a program stored in the storage unit 14 and stores the read program in the memory 92. When the processor 91 executes the program stored in the memory 92, the reference sequence information acquisition device 1 functions as a device including the control unit 11, the input unit 12, the communication unit 13, the storage unit 14, and the output unit 15.

The control unit 11 controls operations of various functional units included in the reference sequence information acquisition device 1. The control unit 11 executes, for example, the reference sequence information acquisition. The control unit 11 records, in the storage unit 14, reference sequence information obtained by executing the reference sequence information acquisition, for example.

The input unit 12 includes an input device such as a mouse, a keyboard, or a touch panel. The input unit 12 may be formed as an interface that connects these input devices to the reference sequence information acquisition device 1. The input unit 12 receives input of various types of information to the reference sequence information acquisition device 1.

The communication unit 13 includes a communication interface for connecting the reference sequence information acquisition device 1 to an external device. The communication unit 13 communicates with the external device via a wired or wireless network. The external device is, for example, the evaluation device 2. The communication unit 13 outputs the reference sequence information to the evaluation device 2 through communication with the evaluation device 2. The external device is, for example, a device that is a transmission source of the first base sequence information and the second base sequence information. The communication unit 13 acquires the first base sequence information and the second base sequence information through communication with the device that is the transmission source of the first base sequence information and the second base sequence information.

The first base sequence information and the second base sequence information do not need to be acquired by the reference sequence information acquisition device 1 via the communication unit 13. The first base sequence information and the second base sequence information may be acquired by the reference sequence information acquisition device 1 by inputting the first base sequence information and the second base sequence information to the input unit 12.

The storage unit 14 is formed using a non-transitory computer readable storage medium device such as a magnetic hard disk device or a semiconductor storage device. The storage unit 14 stores various types of information related to the reference sequence information acquisition device 1. The storage unit 14 stores information input via the input unit 12 or the communication unit 13, such as the first base sequence information and the second base sequence information. The storage unit 14 stores, for example, reference sequence information obtained by executing the reference sequence information acquisition.

The output unit 15 outputs various types of information. The output unit 15 includes a display device such as a cathode ray tube (CRT) display, a liquid crystal display, or an organic electro-luminescence (EL) display. The output unit 15 may be formed as an interface that connects these input devices to the reference sequence information acquisition device 1. The output unit 15 outputs, for example, information input to the input unit 12 or the communication unit 13. The output unit 15 may display, for example, the reference sequence information obtained by executing the reference sequence information acquisition.

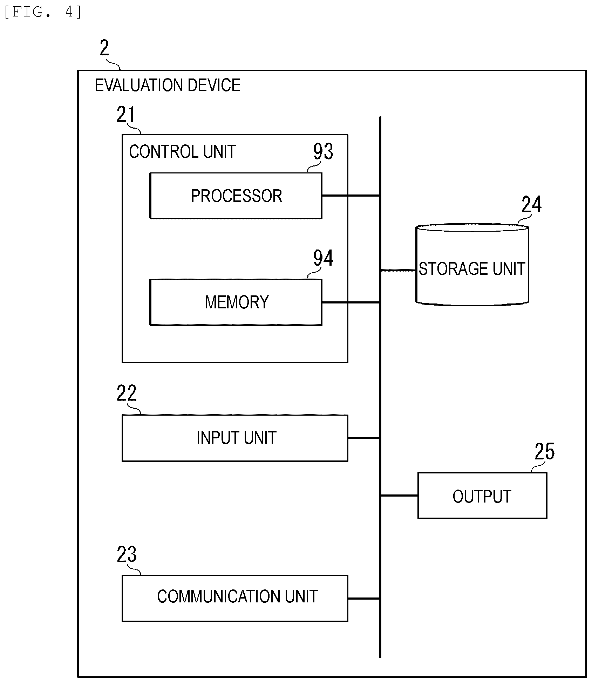

FIG. 4 is a diagram showing an example of a hardware configuration of the evaluation device 2 according to the embodiment. The evaluation device 2 includes a control unit 21 including a processor 93 such as a CPU and a memory 94 which are connected to a bus, and executes a program. The evaluation device 2 functions as a device including a control unit 21, an input unit 22, a communication unit 23, a storage unit 24, and an output unit 25 by executing the program.

More specifically, the processor 93 reads a program stored in the storage unit 24 and stores the read program in the memory 94. When the processor 93 executes the program stored in the memory 94, the evaluation device 2 functions as a device including the control unit 21, the input unit 22, the communication unit 23, the storage unit 24, and the output unit 25.

The control unit 21 controls operations of various functional units included in the evaluation device 2. The control unit 21 executes, for example, evaluation. The control unit 21 records, in the storage unit 24, a result obtained by executing the evaluation, for example.

The input unit 22 includes an input device such as a mouse, a keyboard, or a touch panel. The input unit 22 may be formed as an interface that connects these input devices to the evaluation device 2. The input unit 22 receives input of various types of information to the evaluation device 2.

The communication unit 23 includes a communication interface for connecting the evaluation device 2 to an external device. The communication unit 23 communicates with the external device via a wired or wireless network. The external device is, for example, the reference sequence information acquisition device 1. The communication unit 23 acquires reference sequence information through communication with the reference sequence information acquisition device 1. The external device is, for example, a device that is a transmission source of comparison target sequence information. The communication unit 23 acquires comparison target sequence information through communication with the device that is the transmission source of the comparison target sequence information.

The reference sequence information and the comparison target sequence information do not necessarily have to be acquired by the evaluation device 2 via the communication unit 23. The reference sequence information and the comparison target sequence information may be acquired by the evaluation device 2 by inputting the reference sequence information and the comparison target sequence information to the input unit 22.

The storage unit 24 is formed using a non-temporary computer-readable storage medium device such as a magnetic hard disk device or a semiconductor storage device. The storage unit 24 stores various types of information related to the evaluation device 2. The storage unit 24 stores information input via the input unit 22 or the communication unit 23, such as the reference sequence information, the comparison target sequence information, and the reference sequence information. The storage unit 24 records, for example, a result obtained by executing the evaluation.

The output unit 25 outputs various types of information. The output unit 25 includes a display device such as a CRT display, a liquid crystal display, or an organic EL display. The output unit 25 may be formed as an interface that connects these display devices to the evaluation device 2. The output unit 25 outputs, for example, information input to the input unit 22 or the communication unit 23. The output unit 25 may display, for example, a result obtained by executing the evaluation.

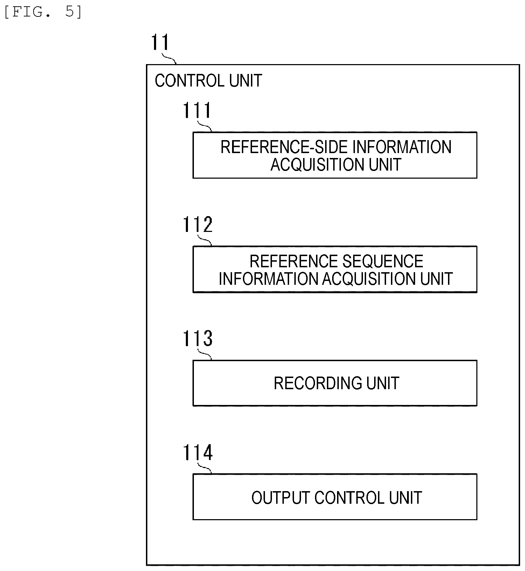

FIG. 5 is a diagram showing an example of a functional configuration of the control unit 11 according to the embodiment. The control unit 11 includes a reference-side information acquisition unit 111, a reference sequence information acquisition unit 112, a recording unit 113, and an output control unit 114.

The reference-side information acquisition unit 111 acquires the first base sequence information and the second base sequence information input to the input unit 12 or the communication unit 13. When the first base sequence information and the second base sequence information have been stored in the storage unit 14 in advance, the reference-side information acquisition unit 111 may acquire the first base sequence information and the second base sequence information by reading the first base sequence information and the second base sequence information from the storage unit 14.

The reference sequence information acquisition unit 112 executes the reference sequence information acquisition. The reference sequence information acquisition unit 112 acquires reference sequence information based on the first base sequence information and the second base sequence information by executing the reference sequence information acquisition.

The recording unit 113 stores various types of information in the storage unit 14. The recording unit 113 records, in the storage unit 14, the first base sequence information and the second base sequence information input to the input unit 12 or the communication unit 13, for example. The recording unit 113 records, for example, the reference sequence information in the storage unit 14. The output control unit 114 controls an operation of the output unit 15. The output unit 15 displays, for example, the first base sequence information and the second base sequence information under the control of the operation by the output control unit 114.

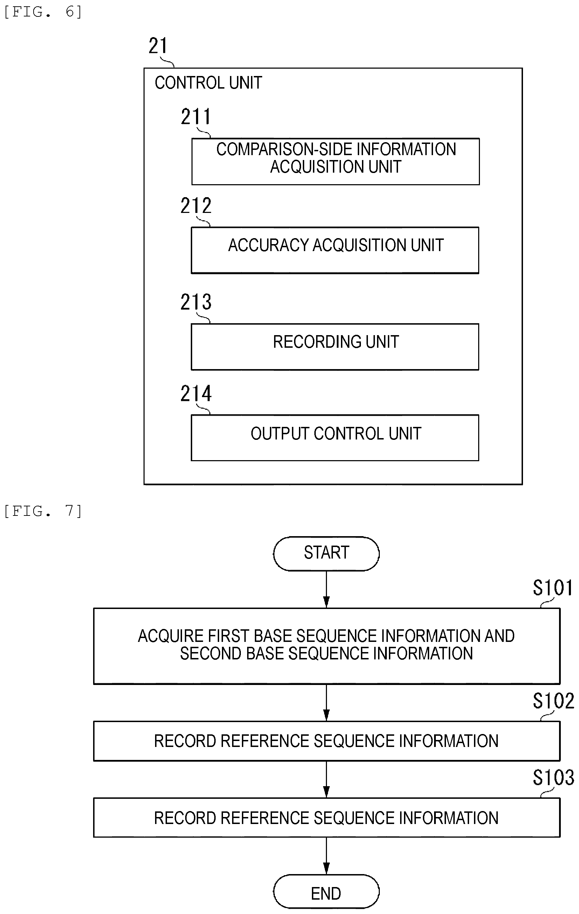

FIG. 6 is a diagram showing an example of a functional configuration of the control unit 21 according to the embodiment. The control unit 21 includes a comparison-side information acquisition unit 211, an accuracy acquisition unit 212, a recording unit 213, and an output control unit 214.

The comparison-side information acquisition unit 211 acquires reference sequence information and comparison target sequence information input to the input unit 22 or the communication unit 23. When the reference sequence information has been stored in the storage unit 24 in advance, the comparison-side information acquisition unit 211 may acquire the reference sequence information by reading the reference sequence information from the storage unit 24.

The accuracy acquisition unit 212 executes evaluation. The accuracy acquisition unit 212 acquires a matching accuracy between a reference sequence and a base sequence of an analysis target by executing the evaluation.

The recording unit 213 stores various types of information in the storage unit 24. The recording unit 213 records, in the storage unit 24, the reference sequence information and the comparison target sequence information input to the input unit 22 or the communication unit 23, for example. The recording unit 213 records, for example, the reference sequence information in the storage unit 24. The output control unit 214 controls an operation of the output unit 25. The output unit 25 displays, for example, the reference sequence information and the comparison target sequence information under the control of the operation by the output control unit 214.

FIG. 7 is a flowchart showing an example of a flow of processing executed by the reference sequence information acquisition device 1 according to the embodiment. The reference-side information acquisition unit 111 acquires the first base sequence information and the second base sequence information (step S101) . Next, the reference sequence information acquisition unit 112 executes the reference sequence information acquisition (step S102). Reference sequence information is acquired by the processing of step S102. Next, the recording unit 213 records the reference sequence information in the storage unit 14 (step S103) .

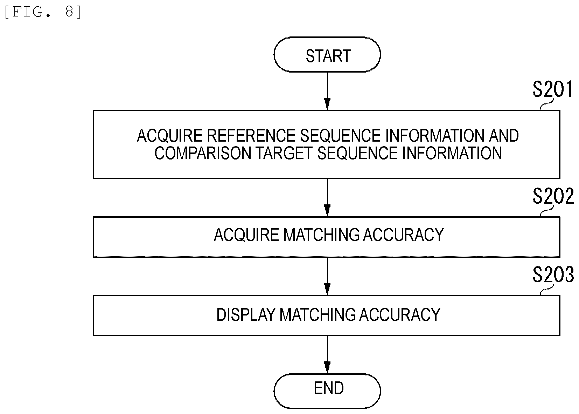

FIG. 8 is a flowchart showing an example of a flow of processing executed by the evaluation device 2 according to the embodiment. The comparison-side information acquisition unit 211 acquires reference sequence information and comparison target sequence information (step S201). Next, the accuracy acquisition unit 212 executes the evaluation (step S202). By the processing of step S202, a matching accuracy between a reference sequence and a base sequence of a comparison target is acquired. Next, the output control unit 214 controls an operation of the output unit 25 to cause the output unit 25 to display the matching accuracy (step S203) .

(Application Example 1)

When it is suspected whether a product distributed under a specific variety name is of that variety, it is possible to perform variety determination of the product by using the genetic information analysis system 100 to obtain a matching accuracy. For example, by acquiring the first base sequence and the second base sequence from a product sample and using a base sequence of the variety as a comparison target sequence, the matching accuracy is acquired using the genetic information analysis system 100. A higher matching accuracy indicates that the product is more similar to the variety. When the matching accuracy is approximately 100% (for example, 96% or more, 97% or more, 98% or more, or 99% or more), the product can be determined as the variety.

(Application Example 2)

When an analysis target sample is considered to be a specific biological variety mixed with other varieties, a mixing ratio of the other varieties in the analysis target sample can be obtained by obtaining the matching accuracy using the genetic information analysis system 100. For example, in the case of granular or powdery products such as cereal products, an amount thereof may be increased by mixing a variety different from a brand name. For example, by acquiring the first base sequence and the second base sequence from a product sample and using a base sequence of the branded grain as a comparison target sequence, a mixing ratio of the non-branded variety can be acquired using the genetic information analysis system 100.

(Application Example 3)

When an individual organism from which an analysis target sample is derived has a plurality of candidate parents, it is possible to perform paternity testing by using the genetic information analysis system 100 to obtain a matching accuracy. For example, the first base sequence and the second base sequence are acquired from the individual organism as an analysis target, and reference sequence information is acquired. A base sequence is acquired from each of the individual organisms of a father candidate and a mother candidate, and a set of these base sequences is acquired as comparison target sequence information. The matching accuracy is acquired by the genetic information analysis system 100 using the reference sequence information and the comparison target sequence information. When the matching accuracy is approximately 100% (for example, 96% or more, 97% or more, 98% or more, or 99% or more), a combination of parent candidates can be determined to be a combination of parents of the individual organisms.

The genetic information analysis system 100 configured in this way evaluates the similarity (that is, a degree of similarity) or a mixing ratio of genetic information between the analysis target and the comparison target by comparing the reference sequence with the base sequence of the comparison target.

As described above, the reference sequence is a base sequence common to the first base sequence and the second base sequence. Thus, the reference sequence is a base sequence that matches between two base sequences even after nucleic acid amplification for preparation of a sequence analysis sample and sequence analysis by a sequencer. Therefore, the reference sequence is a base sequence in which an influence of an amplification error or a read error due to nucleic acid amplification and/or sequence analysis is lower than that of the first base sequence or the second base sequence.

Therefore, the genetic information analysis system 100, which uses the reference sequence to evaluate the similarity of types between the analysis target and the comparison target, can evaluate the similarity or a mixing ratio between the analysis target and the comparison target with a higher accuracy. Therefore, the genetic information analysis system 100 can select highly accurate base sequence data that can withstand the evaluation of similarity or mixing.

(Modification)

The reference sequence is not necessarily a base sequence that is common to two base sequences of the first base sequence and the second base sequence, and may be a base sequence that is common to three or more base sequences obtained by three or more independent sequence acquisition operations from an analysis target sample.

Therefore, in the reference sequence information acquisition, the reference sequence information does not have to be acquired based on only two pieces of analysis sequence information including the first base sequence information and the second base sequence information, and may be acquired based on three or more pieces of analysis sequence information.

The reference sequence information acquisition may be executed repeatedly. In such a case, at least one of the first base sequence and the second base sequence may be the reference sequence obtained by the reference sequence information acquisition executed immediately before. In this way, a reference sequence acquired by the reference sequence information acquisition device 1 may be obtained as a result of repeating the reference sequence information acquisition a predetermined number of times.

The comparison-side information acquisition unit 211 may acquire, via the communication unit 23, comparison target sequence information from a predetermined external device that stores genetic information.

Reference sequence information acquired from a specific analysis target may be recorded in the storage unit 24, and may be used as the comparison target sequence information when the evaluation is performed by acquiring reference sequence information from different analysis targets.

The reference sequence information acquisition device 1 and the evaluation device 2 do not need to be implemented with different housings, and may be implemented as a single device. For example, the control unit 11 may further include the comparison-side information acquisition unit 211 and the accuracy acquisition unit 212, and may execute the reference sequence information acquisition and the evaluation. In such a case, the reference sequence information acquisition device 1 functions as a device that executes not only the reference sequence information acquisition but also the evaluation.

The genetic information analysis system 100, the reference sequence information acquisition device 1, and the evaluation device 2 may be implemented using a plurality of information processing devices communicably connected via a network. In this case, each functional unit included in each of the genetic information analysis system 100, the reference sequence information acquisition device 1, and the evaluation device 2 may be distributed and implemented in the plurality of information processing devices.

All or part of the functions of the genetic information analysis system 100, the reference sequence information acquisition device 1, and the evaluation device 2 may be implemented using hardware such as an application specific integrated circuit (ASIC), a programmable logic device (PLD), or a field programmable gate array (FPGA). The program can be recorded in a computer-readable recording medium. The computer-readable recording medium refers to a storage device such as a portable medium such as a flexible disk, a magneto-optical disk, a ROM, and a CD-ROM, and a hard disk built in a computer system. The program may be transmitted via a telecommunication line.

[Specific Sequence Information Acquisition System]

By using the reference sequence, the same genetic information as the analysis target can be identified as a specific sequence that can be distinguished from other genetic information. For simplification of the description, a case will be described below as an example in which a target whose specific sequence is to be identified is a biological variety.

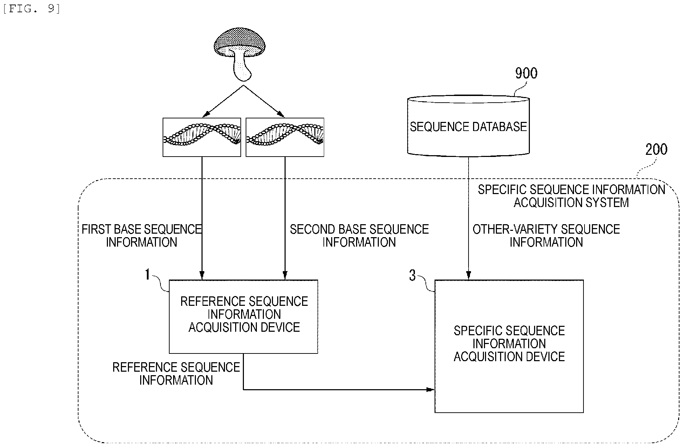

FIG. 9 is a diagram showing an example of a configuration of a specific sequence information acquisition system 200 according to the embodiment. The specific sequence information acquisition system 200 acquires specific sequence information, which is information indicating a specific sequence. The specific sequence is a sequence specifically present in the genetic information included in the analysis target. For example, when the analysis target is a specific variety, the specific sequence is a base sequence capable of specifically detecting the specific variety. That is, the specific sequence is a detection marker capable of detecting genetic information on the specific variety.

The specific sequence information acquisition system 200 includes the reference sequence information acquisition device 1 and a specific sequence information acquisition device 3.

The reference sequence information acquisition device 1 is the same as described above.

The specific sequence information acquisition device 3 executes specific sequence information acquisition. The specific sequence information acquisition is processing for acquiring information on a base sequence specific to the reference sequence (a base sequence that exists only in the reference sequence and does not exist in other-variety sequences) based on the reference sequence information and information (hereinafter referred to as "other-variety sequence information") indicating a base sequence of other variety (hereinafter referred to as "other-variety sequence").

The other-variety sequences are base sequences of genetic information on a variety different from the variety of the analysis target from which the reference sequence is obtained. The other-variety sequences are preferably derived from a plurality of varieties. The other-variety sequences are preferably acquired from a plurality of varieties that are highly related to the variety of the analysis target. Accordingly, it is possible to acquire a specific sequence that can distinguish the variety of the analysis target even from highly related varieties. The other-variety sequences can be acquired, for example, from public information on base sequences. The public information on the base sequence can be obtained from, for example, a gene database (GenBank, DDBJ, or the like) or a document.

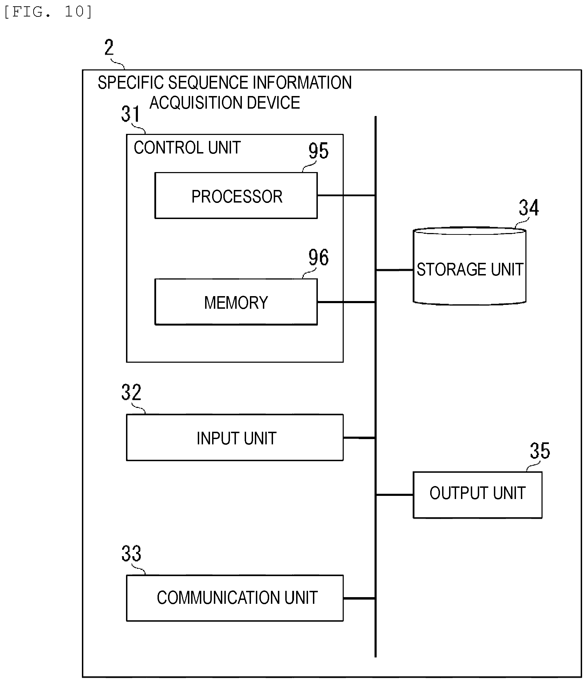

FIG. 10 is a diagram showing an example of a hardware configuration of the specific sequence information acquisition device 3 according to the embodiment. The specific sequence information acquisition device 3 includes the control unit 21 including a processor 95 such as a CPU and a memory 96 which are connected to a bus, and executes a program. The evaluation device 2 functions as a device including the control unit 21, the input unit 22, the communication unit 23, the storage unit 24, and the output unit 25 by executing the program.

More specifically, the processor 95 reads a program stored in the storage unit 34 and causes the memory 96 to store the read program. By the processor 95 executing the program stored in the memory 96, the specific sequence information acquisition device 3 functions as a device including a control unit 31, an input unit 32, a communication unit 33, a storage unit 34, and the output unit 25.

The control unit 31 controls operations of various functional units included in the specific sequence information acquisition device 3. The control unit 31 executes, for example, the specific sequence information acquisition. The control unit 31 records, in the storage unit 34, a result obtained by executing the specific sequence information acquisition, for example.

The input unit 32 includes an input device such as a mouse, a keyboard, or a touch panel. The input unit 32 may be formed as an interface that connects these input devices to the specific sequence information acquisition device 3. The input unit 32 receives input of various types of information to the specific sequence information acquisition device 3.

The communication unit 33 includes a communication interface for connecting the specific sequence information acquisition device 3 to an external device. The communication unit 33 communicates with the external device via a wired or wireless network. The external device is, for example, the reference sequence information acquisition device 1. The communication unit 33 acquires reference sequence information through communication with the reference sequence information acquisition device 1. The external device is, for example, a device that is a transmission source of the other-variety sequence information. The communication unit 33 acquires other-variety sequence information through communication with the device that is the transmission source of the other-variety sequence information. A sequence database 900 in FIG. 9 is an example of a transmission source of other-variety sequence information.

The reference sequence information and the other-variety sequence information do not necessarily have to be acquired by the specific sequence information acquisition device 3 via the communication unit 33. The reference sequence information and the other-variety sequence information may be acquired by the specific sequence information acquisition device 3 by inputting the reference sequence information and the other-variety sequence information to the input unit 32.

The storage unit 34 is formed using a non-transitory computer readable storage medium device such as a magnetic hard disk device or a semiconductor storage device. The storage unit 34 stores various types of information related to the specific sequence information acquisition device 3. The storage unit 34 stores information input via the input unit 32 or the communication unit 33, such as the reference sequence information, other-variety sequence information, and the reference sequence information. The storage unit 34 records, for example, a result obtained by executing the specific sequence information acquisition.

The output unit 35 outputs various types of information. The output unit 35 includes a display device such as a CRT display, a liquid crystal display, or an organic EL display. The output unit 35 may be formed as an interface that connects these display devices to the specific sequence information acquisition device 3. The output unit 35 outputs, for example, information input to the input unit 32 or the communication unit 33. The output unit 35 may display, for example, a result obtained by executing the specific sequence information acquisition.

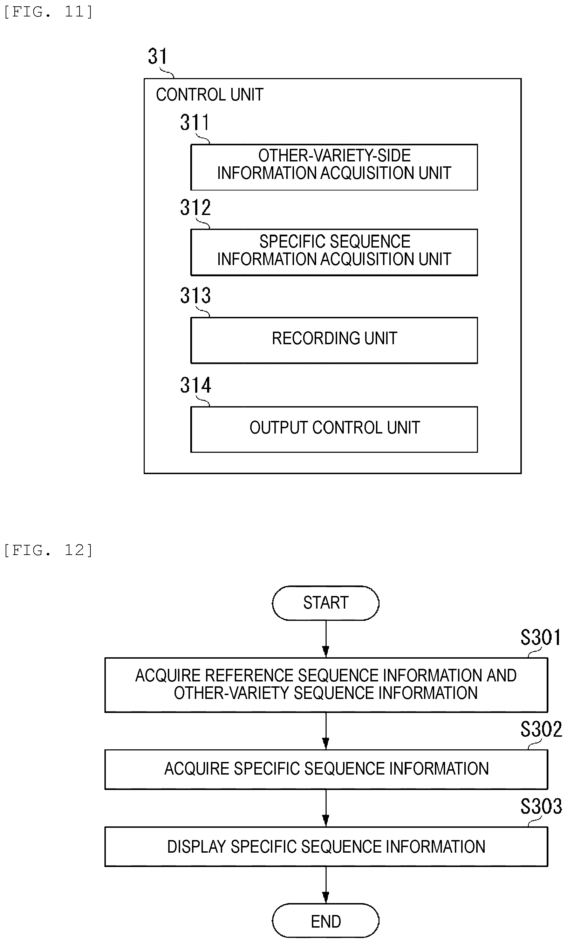

FIG. 11 is a diagram showing an example of a functional configuration of the control unit 31 according to the embodiment. The control unit 31 includes other-variety-side information acquisition unit 311, a specific sequence information acquisition unit 312, a recording unit 313, and an output control unit 314.

The other-variety-side information acquisition unit 311 acquires reference sequence information and other-variety sequence information input to the input unit 32 or the communication unit 33. When the reference sequence information has been stored in the storage unit 34 in advance, the other-variety-side information acquisition unit 311 may acquire the reference sequence information by reading the reference sequence information from the storage unit 34.

The specific sequence information acquisition unit 312 executes specific sequence information acquisition. The specific sequence information acquisition unit 312 acquires specific sequence information indicating a specific sequence that is only present in the reference sequence and not present in the other-variety sequences by executing the specific sequence information acquisition.

The recording unit 313 stores various types of information in the storage unit 34. The recording unit 313 records, in the storage unit 34, the reference sequence information and the other-variety sequence information input to the input unit 32 or the communication unit 33, for example. The recording unit 313 records, for example, the reference sequence information in the storage unit 34. The output control unit 314 controls an operation of the output unit 35. The output unit 35 displays, for example, the reference sequence information and the other-variety sequence information under the control of the operation by the output control unit 314.

FIG. 12 is a flowchart showing an example of a flow of processing executed by the specific sequence information acquisition device 3 according to the embodiment. The other-variety-side information acquisition unit 311 acquires reference sequence information and other-variety sequence information (step S301). Next, the specific sequence information acquisition unit 312 executes specific sequence information acquisition (step S302). By the process of step S302, specific sequence information indicating a specific sequence capable of distinguishing the reference sequence from the other-variety sequence is acquired. Next, the output control unit 314 controls an operation of the output unit 35 to cause the output unit 35 to display the specific sequence information (step S303).

The specific sequence information acquisition system 200 configured in this manner obtains specific sequence information indicating a specific sequence, which is a base sequence specifically present in the analysis target, by comparing the reference sequence with the other-variety sequence.

As described above, the reference sequence is a base sequence in which an influence of an amplification error or a read error due to nucleic acid amplification and/or sequence analysis is lower than that of the first base sequence or the second base sequence. Therefore, the specific sequence information acquisition system 200, which acquires the specific sequence information using the reference sequence, can identify a specific sequence with higher accuracy. Therefore, the specific sequence acquired by the specific sequence information acquisition system 200 can provide an analysis target detection technique with high reliability.

(Modification)

The other-variety-side information acquisition unit 311 may acquire, via the communication unit 33, other-variety sequence information from a predetermined external device that stores genetic information on the other varieties.

The reference sequence information acquisition device 1 and the specific sequence information acquisition device 3 do not need to be mounted on different housings, and may be implemented as a single device. For example, the control unit 11 may further include the other-variety-side information acquisition unit 311 and the specific sequence information acquisition unit 312, and may execute the reference sequence information acquisition and specific sequence identification. In such a case, the reference sequence information acquisition device 1 functions as a device that executes not only the reference sequence information acquisition but also the specific sequence identification.

The specific sequence information acquisition system 200, the reference sequence information acquisition device 1, and the specific sequence information acquisition device 3 may be implemented using a plurality of information processing devices communicably connected via a network. In this case, each functional unit included in each of the specific sequence information acquisition system 200, the reference sequence information acquisition device 1, and the specific sequence information acquisition device 3 may be distributed and implemented in the plurality of information processing devices.

All or part of the functions of the specific sequence information acquisition system 200, the reference sequence information acquisition device 1, and the specific sequence information acquisition device 3 may be implemented using hardware such as an application specific integrated circuit (ASIC), a programmable logic device (PLD), or a field programmable gate array (FPGA). The program can be recorded in a computer-readable recording medium. The computer-readable recording medium refers to a storage device such as a portable medium such as a flexible disk, a magnetooptical disk, a ROM, and a CD-ROM, and a hard disk built in a computer system. The program may be transmitted via a telecommunication line.

[Genetic Information Analysis Method, Reference Sequence Information Acquisition Method, and Specific Sequence Information Acquisition Method]

The invention also provides a genetic information analysis method. The genetic information analysis method includes: a step of acquiring comparison target sequence information indicating a comparison target sequence which is a base sequence of genetic information on a comparison target; and a step of acquiring an accuracy (matching accuracy) that the genetic information on the comparison target is the same as genetic information on an analysis target, based on the comparison target sequence information and reference sequence information indicating a reference sequence selected from base sequences common to two or more independently acquired base sequences of the genetic information on the analysis target. The genetic information analysis method may be performed by the genetic information analysis system 100. Alternatively, the reference sequence information and the comparison target sequence information may be compared with each other using software or the like attached to the next-generation sequencer, thereby detecting a reference sequence matching the comparison target sequence to acquire the matching accuracy.

The invention also provides a reference sequence information acquisition method. The reference sequence information acquisition method includes a step of acquiring reference sequence information indicating a reference sequence selected from base sequences common to two or more independently acquired base sequences of genetic information on an analysis target. The step may be performed by the reference sequence information acquisition device 1 executing the reference sequence information acquisition. Alternatively, the two or more base sequences may be compared with each other using software or the like attached to the next-generation sequencer to identify a common base sequence and acquire reference sequence information.

The invention also provides a specific sequence information acquisition method. The specific sequence information acquisition method includes: a step of acquiring reference sequence information indicating a reference sequence selected from base sequences common to two or more independently acquired base sequences of genetic information on an analysis target; and a step of acquiring, from the reference sequence information, specific sequence information indicating a base sequence that is specifically present in the reference sequence. The specific sequence information acquisition method may be performed by the specific sequence information acquisition system 300. Alternatively, a program or the like implementing a known sequence alignment algorithm such as basic local alignment search tool (BLAST) may be used to compare the reference sequence information with other-variety sequence information, thereby detecting a specific sequence present only in the reference sequence and acquiring specific sequence information.

[Method for Producing Detection Nucleic Acid]

A detection nucleic acid that specifically detects genetic information on an analysis target can be produced based on specific sequence information acquired by the specific sequence information acquisition system 200 or the specific sequence information acquisition method. The detection nucleic acid can be produced by a known nucleic acid synthesis method such as phosphoramidite method. The detection nucleic acid may contain a specific sequence or may contain a complementary sequence of the specific sequence. The detection nucleic acid can be used as a primer, a probe, or the like for detecting the genetic information on the analysis target. The detection nucleic acid may be DNA, RNA, or a mixture of DNA and RNA. The detection nucleic acid is not limited to a natural nucleic acid, and may include an artificial nucleic acid. Artificial nucleic acid means an artificially synthesized compound having a function similar to that of nucleic acid. Examples of the artificial nucleic acid include, but are not limited to, LNA, BNA, and PNA.

Examples

Hereinafter, the invention will be described in detail with reference to examples, but the invention is not limited to these examples.

(a) Acquisition of Base Sequence Data

In the examples, base sequence data was obtained by MIG-seq using the next-generation sequencer (NGS). However, a method for acquiring the base sequence data is not limited thereto.

(Preparation of Sequence Analysis Sample)

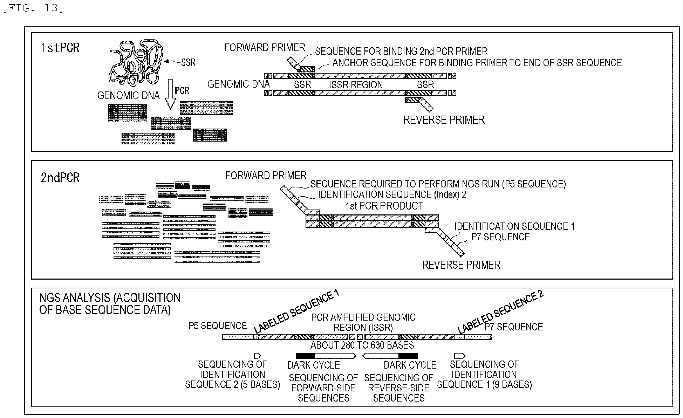

In order to prepare a sequence analysis sample for acquiring the first base sequence, DNA (first base sequence sample) was extracted from the analysis target sample.
In order to prepare a sequence analysis sample for acquiring the second base sequence, DNA (second base sequence sample) was extracted from the analysis target sample by an operation independent of an operation for acquiring the first base sequence.
In order to prepare a sequence analysis sample for acquiring a comparison target sequence, DNA (comparison target sequence sample) was extracted from a comparison target sample.
For each sample, a DNA fragment group (library) was constructed as a sequence analysis sample for the next-generation sequencer. The library was constructed using MIG-seq (see FIG. 13: modified based on Yoshihisa Suyama (2019), Use of MIG-seq in forest genetic and breeding studies. Forest Genetics and Breeding 8(2) : 85-89) in accordance with a method of Suyama & Matsuki (2015). However, an annealing temperature for a 1st PCR was 38°C. In addition, addition of labeled sequences (indexes) in a 2nd PCR step was performed on both sides.
As the 1st PCR (amplification of ISSR region), PCR amplification between inter-simple sequence repeat (ISSR) regions was performed using set-1 primers of Suyama & Matsuki (2015). Primers used in the 1st PCR are shown in Table 1.

[Table 1]

| Sequence Name | Sequence (5'-3') | Sequence Number |
|---|---|---|
| Forward primers: | (Tail + **anchor: CTG**) + SSR + **anchor** | |
| (ACT)$_4$TG-f | CGCTCTTCCGATCT**CTG**ACTACTACTACT**TG** | 1 |
| (CTA)$_4$TG-f | CGCTCTTCCGATCT**CTG**CTACTACTACTA**TG** | 2 |
| (TTG)$_4$AC-f | CGCTCTTCCGATCT**CTG**TTGTTGTTGTTG**AC** | 3 |
| (GTT)$_4$CC-f | CGCTCTTCCGATCT**CTG**GTTGTTGTTGTT**CC** | 4 |
| (GTT)$_4$TC-f | CGCTCTTCCGATCT**CTG**GTTGTTGTTGTT**TC** | 5 |
| (GTG)$_4$AC-f | CGCTCTTCCGATCT**CTG**GTGGTGGTGGTG**AC** | 6 |
| (GT)$_6$TC-f | CGCTCTTCCGATCT**CTG**GTGTGTGTGTGT**TC** | 7 |
| (TG)$_6$AC-f | CGCTCTTCCGATCT**CTG**TGTGTGTGTGTG**AC** | 8 |
| Reverse primers: | (Tail + **anchor: GAC**) + SSR + **anchor** | |
| (ACT)$_4$TG-r | TGCTCTTCCGATCT**GAC**ACTACTACTACT**TG** | 9 |
| (CTA)$_4$TG-r | TGCTCTTCCGATCT**GAC**CTACTACTACTA**TG** | 10 |

(continued)

| Sequence Name | Sequence (5'-3') | Sequence Number |
|---|---|---|
| (TTG)$_4$AC-r | TGCTCTTCCGATCT**GAC**TTGTTGTTGTTG**AC** | 11 |
| (GTT)$_4$CC-r | TGCTCTTCCGATCT**GAC**GTTGTTGTTGTT**CC** | 12 |
| (GTT)$_4$TC-r | TGCTCTTCCGATCT**GAC**GTTGTTGTTGTT**TC** | 13 |
| (GTG)$_4$AC-r | TGCTCTTCCGATCT**GAC**GTGGTGGTGGTG**AC** | 14 |
| (GT)$_6$TC-r | TGCTCTTCCGATCT**GAC**GTGTGTGTGTGT**TC** | 15 |
| (TG)$_6$AC-r | TGCTCTTCCGATCT**GAC**TGTGTGTGTGTG**AC** | 16 |

As a reagent for the 1st PCR, Multiplex PCR Assay Kit Ver. 2 (Takara Bio) was used. Reaction solution composition and reaction conditions are shown in Tables 2 and 3, respectively.

[Table 2]

| | |
|---|---|
| Template DNA | 1.00 $\mu$L |
| 2x Multiplex PCR Buffer | 3.00 $\mu$L |
| 1st PCR primers mixture (Concentration: each 20 $\mu$M) | 0.96 $\mu$L (Final concentration: each 0.2 $\mu$M) |
| Multiplex PCR Enzyme mix | 0.03 $\mu$L |
| ddH$_2$0 | 1.01 $\mu$l |
| Total | 6.00 $\mu$L |

[Table 3]

| 1 Cycle | |
|---|---|
| 94°C | 1 min |
| 25 Cycle | |
| 94°C | 30 sec |
| 38°C | 1 min |
| 72°C | 1 min |
| 1 Cycle | |
| 72°C | 10 min |

Bead purification was performed using AMPure XP (BECKMAN COULTER) for the purpose of removing contaminants remaining in a 1st PCR product, selecting a size (remove short fragments of 200 bp or less), and averaging concentrations between samples. A purification procedure follows a standard protocol of the product.

The 2nd PCR was performed under reaction conditions shown in Table 5 using a reaction solution having a composition shown in Table 4 for the purpose of adding labeled sequences (nine bases at a Read-1 side and five bases at a Read-2 side) for sample identification and adapter sequences (P5 and P7 sequence) for an Illumina MiSeq Sequencer (Illumina). Primer sequences used for the 2nd PCR are shown below (labeled sequences differ from sample to sample and thus are shown as n sequences).

Read-1 side (forward primer):

5'-AATGATACGGCGACCACCGAGATCTACACnnnnnACACTCTTTCCCTAC

ACGACGCTCTTCCGATCTCTG-3' (SEQ ID NO: 17)

Read-2 side (reverse primer):

5'-CAAGCAGAAGACGGCATACGAGATnnnnnnnnnnGTGACTGGAGTTCAGA

CGTGTGCTCTTCCGATCTGAC-3' (SEQ ID NO: 18)

[Table 4]

| | |
|---|---|
| Purified 1st PCR product | 1.60 μL |
| 2nd PCR forward primer (Concentration: 1 μM) | 1.20 μL (Final concentration: each 0.2 μM) |
| 2nd PCR reverse primer (Concentration: 1 μM) | 1.20 uL (Final concentration: each 0.2 μM) |
| 5 × PrimeSTAR GXL Buffer | 1.20 μL |
| dNTP mixture | 0.48 μL |
| PrimerSTAR GXL polymerase | 0.12 μL |
| ddH$_2$0 | 0.20 μL |
| Total | 6.00 μL |

[Table 5]

| 12 Cycle | |
|---|---|
| 98°C | 10 sec |
| 54°C | 15 sec |
| 68°C | 1 min |

Bead purification was performed using AMPure XP (BECKMAN COULTER) for the purpose of removing contaminants remaining in a 2nd PCR product and selecting a size. Unlike the 1st product, a labeled sequence for identification was added to the 2nd PCR product of each of the samples (the first base sequence sample, the second base sequence sample, and the comparison target sequence sample). Therefore, purification of the 2nd PCR product was performed using an equivalent mixture of each sample. The selected size was set to 400 bp or more so that the base sequences of the Read-1 side and the Read-2 side do not overlap each other.

In order to grasp a rough molar concentration of the library that had been subjected to contaminant removal and size selection, a DNA weight concentration was measured using a Qubit 2.0 fluorometer (Invitrogen, Life Technologies), and the molar concentration was calculated using the following equation. An average fragment size (base sequence length) was 550 bp measured using a MultiNA (Shimadzu Corporation), which is a microchip electrophoresis apparatus, and an average molecular weight was 660.

[Equation 1]

$$\text{Molar concentration } (nM) = \frac{\text{Weight concentration} \left(\frac{ng}{uL}\right) \times 10^6}{550(bp) \times 660}$$

The accurate molar concentration measurement of the library was performed by a CFX Connect™ real-time PCR analysis system (Bio-Rad) using Library Quantification Kit (Takara Bio). As a preparation for the measurement, three stages of diluents for measurement were prepared based on a molar concentration calculated from a measured value of Qubit such that the molar concentration was within a range of a molar concentration of a standard sample (0.01 pM to 10 pM). The reaction solution composition and the reaction conditions followed a standard protocol of Library Quantification Kit (TaKaRa Bio), and the standard sample and the diluents were all measured in three repetitions. The molar concentration using the CFX Connect™ real-time PCR analysis system was set to be calculated assuming that the average fragment size was 447 bp. Therefore, an actual molar concentration of the library was calculated based on an average value of a total of 9 samples (three stages of diluents × three repetitions) measured using the following

equation.

[Equation 2]

$$\text{Molar concentration}\,(nM) = \frac{\text{SQ(Starting Quantity)} \times \left(\text{Dilution ratio}\right)}{1000} \times \frac{447(\text{bp})}{550(\text{bp})}$$

The validation of the library was adjusted as a 12 pM library containing 2% of Phi X according to a standard protocol of Illumina.

(Sequence Analysis)

Next-generation sequencing was performed with an Illumina MiSeq Sequencer (Illumina) using MiSeq Reagent kit v2 (300 cycle, Illumina). The sequencing was performed at Paired End when reading the library from both ends, and the sequencing reaction was performed for 117 cycles on each side. Originally, the sequencing reaction can be performed for 150 cycles on each side, but since a quality value (QV) decreases as the number of cycles increases, the sequencing reaction is performed only up to 117 cycles on each side in the examples. In addition, since 17 bases at both ends of the library constructed by MIG-seq are primer sequences of the 1st PCR, which has a biased base balance, Dark Cycle was set for 17 bases from the beginning of reading on the Read-1 side and Read-2 side, and sequence signals (images) were not acquired. That is, the sequence signals were acquired for 100 cycles on each side and 200 cycles on both sides.

(Purification of Sequence Data)

The sequencing data obtained as described above was analyzed as follows. First, as a first step, file sorting for each sample and sequence information purification (extraction of base sequence data used for identification) were performed. Specifically, the software bcl2fastq (Illumina) was used to generate Fastq files (files storing a base sequence read by sequencing and accuracy information (QV value) on each base) based on the acquired signal information on each cycle, and to sort (demultiplex) the Fastq files based on the labeled sequence for sample identification. As an option setting at that time, an allowable number of mismatches of the labeled sequence was changed from 1, which is a default value, to 0. When the labeled sequence contains at least one base having a QV value of 30 or less, Fastq files on the Read-1 side and the Read-2 side obtained from the same cluster as that of the labeled sequence were removed by a self-made program.

As a second step, quality filtering was performed on each base sequence data stored in a Multi-Fastq file generated for each sample by using software Trimmomatic version 0.32 (Anthony et al. 2014). A detailed procedure will be shown below.

Leading and trailing bases of read sequences often have low QV values, and thus were removed (a sequence length after removal is 98 bases on each side).

Data on base sequences containing a short base sequence of 98 bases or less or an adapter sequence was removed. A parameter of a Sliding window was changed from a default value of 4: 15 to 4: 30 to remove (base sequences having a low QV value more strictly base sequence data thereof was removed when an average QV value of four consecutive bases was less than 30).

As a third step, a sequence on the Read-1 side and a sequence on the Read-2 side after passing through the quality filtering were combined for each same cluster to generate base sequence data on a total of 196 bases. However, when only the base sequence data on one side passed a criterion of the quality filtering, the base sequence data on both sides was removed.

(b) Acquisition of Comparison Target Sequence Information

With respect to a data set obtained from a comparison target sequence acquisition sample, the base sequence data passed through the quality filtering was integrated between two or more data sets obtained from the same sample, and a more exhaustive base sequence data set was generated as a base sequence data set of the comparison target sequence information. The more exhaustive base sequence data set was used as the comparison target sequence information. The number of base sequences of the exhaustive base sequence data set is equal to the number of base sequences after the filtering. In general, this base sequence data set includes a large number of base sequence data completely matching in a sample or between repetitions, but the base sequence data was simply integrated and handled when an amount of data thereof is not so large as to affect a data processing speed. In this example, as the base sequence data set of the comparison target sequence information, one data set obtained by integrating two or more data sets obtained from the same sample was used, but one data set may be used as it is.

(c) Acquisition of Reference Sequence Information

With respect to a data set obtained from the first base sequence acquisition sample and the second base sequence acquisition sample, a base sequence in which 196 bases completely match, including a complementary sequence, for each base sequence passed through the quality filtering was collected for each sample. Next, the number of reads (number of times completely the same base sequence was read) was counted for each of the collected base sequences, and base sequence data whose number of reads was less than 10 in each sample was removed as data having a low reliability. Finally, only the base sequence data completely matched between two data sets including the data set obtained from the first base sequence acquisition sample and the data set obtained from the second base sequence acquisition sample was extracted, and a data set of the reference sequence information was generated. In the examples, the reference sequence information was acquired using two samples including the first base sequence sample and the second base sequence acquisition sample, but the reference sequence information may be acquired using three or more samples.

(d) Acquisition of Matching Accuracy

The data set of the reference sequence information was compared with the data set of the comparison target sequence information obtained as described above to search for base sequences in which the reference sequence completely matches in the comparison target sequence, and the number thereof was obtained. Next, a degree of similarity (similarity and degree of matching) between the samples was calculated using the following Formula (1).
[Equation 3]

$$\text{Matching accuracy (\%)} = \frac{\text{Number of base sequences in reference sequence information that completely match data set of comparison target sequence information}}{\text{Total number of base sequences in reference sequence information}} \times 100 \quad \text{...(1)}$$

(e) Acquisition of Specific Sequence Information

The reference sequence information was searched for a base sequence specific to the analysis target, and primers for PCR were designed. In order to examine the effectiveness of the obtained PCR primers, PCR amplification was performed by Fast Cycling PCR Kit (Qiagen) using, as a template, DNA samples of another sample and a target sample as comparison targets, and the presence or absence of the amplification or base sequence information on an amplification sequence was examined. Reaction solution composition and conditions of Fast Cycling PCR are shown in Tables 6 and 7, respectively.

[Table 6]

| Template DNA | 1.00 μL |
|---|---|
| Fast PCR Master Mix | 3.00 μL |
| Forward PCR primer (Concentration: 20 μM) | 0.15 μL (Final concentration: 0.5 μM) |
| Reverse PCR primer (Concentration: 1 μM) | 0.15 μL (Final concentration: 0.5 μM) |
| ddH$_2$0 | 1.70 μL |
| Total | 6.00 μL |

[Table 7]

| 1 Cycle | |
|---|---|
| 95°C | 5 min |
| 25 Cycle | |
| 96°C | 5 sec |

(continued)

| 25 Cycle | |
|---|---|
| 55°C | 5 sec |
| 68°C | 15 sec |
| 1 Cycle | |
| 72°C | 1 min |

<Example 1: Genome Similarity Analysis of Shiitake Mushroom Variety Samples>

Eight varieties of edible shiitake mushrooms were evaluated for similarity. In this example, closely related variety pairs that are thought to be difficult to identify were purposely included to confirm a level of accuracy in identifying differences within varieties and between varieties. Two samples having known variety names were used as samples for a simulated variety testing to perform variety identification between the samples.

(1) Selection of Test Strain Sample

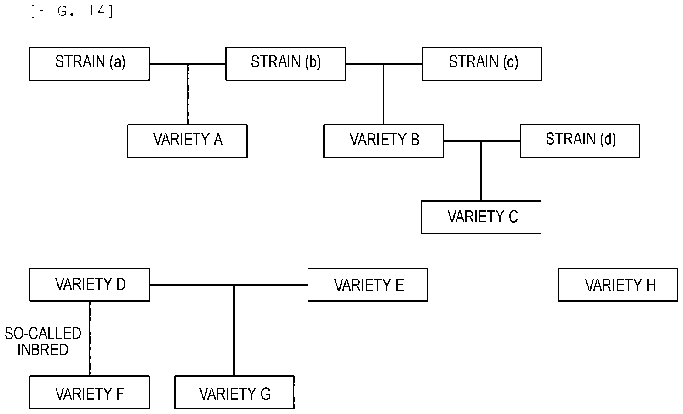

Varieties A to H having clear familial (crossing) correlations at the time of variety production were selected (see FIG. 14). The varieties A to H include, as closely-related varieties, varieties A and B having one common parent, varieties B and C in a parent-child correlation, varieties G, D, and E in a parent-child correlation (crossing parents of G are D and E), and a variety D which is a so-called inbred (crossing breeding of monokaryon mycelium and dikaryon mycelium) parent and F which is a child thereof. For a simulated variety testing, two samples (C-01 and C-02) of the dikaryon mycelium of the variety C, which had been subcultured in a place different from a seeding company that developed the variety, were collected. That is, eight samples of different varieties (the varieties A to H) and two samples for simulated variety testing (the variety C) were used as inspection targets.

(2) DNA extraction

The hyphae were scraped off from each collected mycelium, and DNA was extracted using modified CTAB (Murray and Thompson 1980, Saghai-Maroof et al. 1986).

(3) Acquisition of Reference Sequence Information and Comparison Target Sequence Information

In accordance with (a) to (c) above, the reference sequence information and the comparison target sequence information were acquired. The variety C (C-01, C-02) and the variety D were used to acquire the reference sequence information. The numbers of reference sequences obtained from C-01, C-02, and the variety D were 2,091, 948, and 1,658, respectively. The varieties A, B, E, F, G, and H were used to acquire the comparison target sequence information. The numbers of comparison target sequences were 407,322 to 1,612,254.

(4) Genome Similarity between Varieties

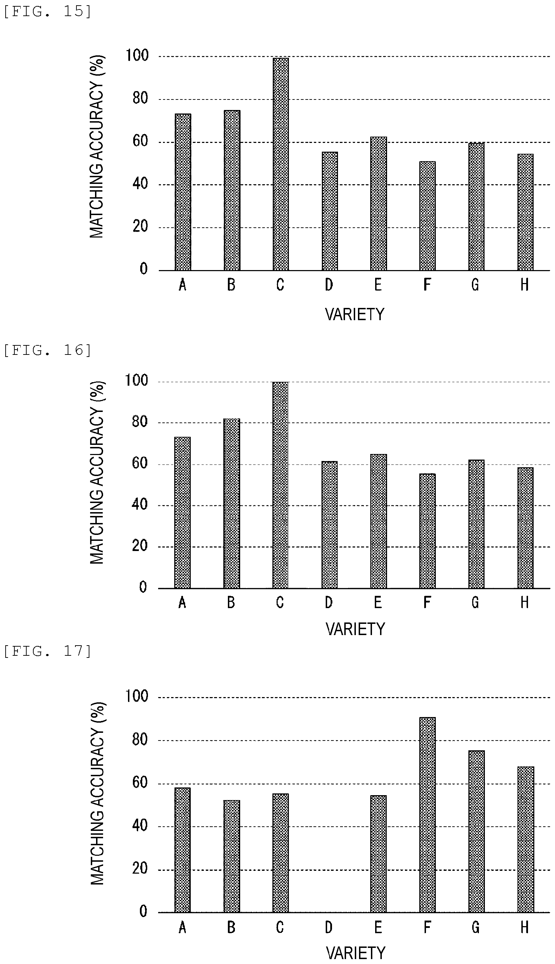

For eight varieties of samples having clear familial correlations, the similarity of the genomes of various varieties with respect to each variety was calculated as the matching accuracy by the above Formula (1).
First, when the matching accuracy of C-01 and C-02, which are samples for the simulated variety testing, was calculated for the variety C. Values thereof were close to 100%, and were 99.3% (FIG. 15: C-01) and 99.9% (FIG. 16: C-02), respectively. This result strongly indicates that C-01 and C-02, which are the samples for the simulated variety testing, are the variety C, which is a correct variety testing result. On the other hand, values of the similarity of C-01 and C-02 to the varieties other than the variety C were in a range of 51.1% to 82.2%. That is, by indicating that the similarity with another variety is clearly low, it was shown that C-01 and C-02, which are the samples for the simulated variety testing, are none of the varieties A, B, and D to H.
As closely related varieties that are considered impossible to be identified by a method in the related art, the matching accuracy with the variety D which is an inbred parent as a reference side and the variety F which is a child of the variety D as a comparison target side was calculated. As a result, the matching accuracy was a relatively high value reflecting the similarity of the genome, but the matching accuracy (90.7%) was clearly lower than a matching accuracy between the same variety described above (almost 100%), and the variety D and the variety F can be identified as different varieties (FIG. 17). The matching accuracy between other closely related varieties in a parent-child correlation was 85.2% at maximum, and thus the varieties can be distinguished clearly (FIG. 17).

<Example 2: Production of Shiitake Mushroom Variety-specific Identification Marker>

Using the base sequence data obtained in Example 1, a target-specific identification marker was produced. That is, as a target of a method of simply comparing a genome of a sample with a genome of a target variety, the variety D was selected from the above.

(1) Identification of Base Sequence Specific to Variety D and Selection of Primers

The following primer set was selected by searching for a base sequence specific to the variety D and using information such as the presence or absence of non-specific amplification and amplification efficiency as criteria for determination.

Forward side
ATGTATAACCTATCCCACC (SEQ ID NO: 19)
Reverse side
TTGACGATAATTTTGCTGGG (SEQ ID NO: 20)

(2) PCR Amplification with Variety-specific Primers and Confirmation of Amplified Product

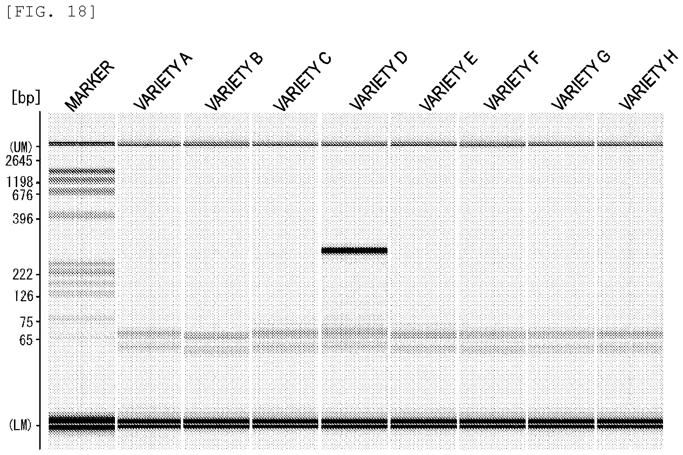

According to the above (e), PCR amplification was performed in the DNA samples prepared from the varieties A to H (see FIG. 14) using the above primer set specific to the variety D. As a result, among the varieties as the inspection target, an amplified product was detected only with the variety D which was set as the target variety (FIG. 18). As a result, it can be said that a marker capable of easily identifying only the variety D by PCR was able to be produced in at least the target varieties.

<Example 3: Paternity testing of Sea Cucumber>

(1) Inspection Target

20 male sea cucumbers (F01 to F20) and 11 female sea cucumbers (M01 to M11) were reared and bred in the same tank. 10 child individuals (O-01 to O-10) obtained by breeding were set as inspection targets to perform the paternity testing.

(2) DNA Extraction

Cells were collected from the 20 male individuals (F01 to F20), the 11 female individuals (M01 to M11), and the 10 child individuals (O-01 to O-10), and DNA was extracted using modified CTAB (Murray and Thompson 1980, Saghai-Maroof et al. 1986).

(3) Acquisition of Reference Sequence Information and Comparison Target Sequence Information

In accordance with (a) to (c) above, the reference sequence information and the comparison target sequence information were acquired. The 10 child individuals (O-01 to O-10) were used to acquire the reference sequence information. The number of reads of the reference sequence information acquired from the child individuals (O-01 to O-10) is shown in Tables 8 to 10.
The 20 male individuals (F01 to F20) and the 11 female individuals (M01 to M11) were used to acquire the comparison target sequence information. The 20 male individuals (F01 to F20) and the 11 female individuals (M01 to M11) were combined to prepare 220 combinations of parent candidates. A set of comparison target sequence information acquired from the male and female individuals of the parent candidate combination was used as a comparison target sequence information data set for the paternity testing. The numbers of reads of the comparison target sequence information data set were 220,000 to 320,000.

(4) Paternity testing

For each of the reference sequence information acquired from the 10 child individuals (O-01to O-10), the matching accuracy with the comparison target sequence information data set of 220 combinations of parent candidates was calculated by the above formula (1). Tables 8 to 10 show combinations of the top 10 parent candidates with the highest matching accuracy for each child individual.

[Table 8]

| Child Individuals | O-01 | | O-02 | | O-03 | | O-04 | |
|---|---|---|---|---|---|---|---|---|
| Number of Reads | 370 | | 361 | | 361 | | 360 | |
| Rank | Parent Candidate | Matching Accuracy (%) | Parent Candidate | Matching Accuracy (%) | Parent Candidate | Matching Accuracy (%) | Parent Candidate | Matching Accuracy (%) |
| 1 | F05-M04 | 100.00 | F05-M10 | 100.00 | F05-M11 | 100.00 | F05-M06 | 100.00 |
| 2 | F08-M04 | 88.11 | F08-M10 | 88.92 | F08-M11 | 85.60 | F08-M06 | 89.17 |
| 3 | F16-M04 | 87.84 | F16-M10 | 86.43 | F05-M03 | 82.83 | F16-M06 | 86.67 |
| 4 | F 15-M04 | 83.24 | F12-M10 | 84.49 | F16-M11 | 81.99 | F04-M06 | 83.89 |
| 5 | F04-M04 | 82.97 | F03-M10 | 82.83 | F05-M08 | 81.72 | F05-M01 | 83.89 |
| 6 | F12-M04 | 82.97 | F04-M10 | 82.83 | F05-M10 | 81.44 | F17-M06 | 83.89 |
| 7 | F03-M04 | 81.89 | F06-M10 | 82.55 | F05-M02 | 80.89 | F11-M06 | 83.61 |
| 8 | F11-M04 | 81.89 | F11-M10 | 82.55 | F04-M11 | 80.61 | F05-M07 | 83.33 |
| 9 | F18-M04 | 81.89 | F09-M10 | 82.27 | F05-M07 | 80.33 | F05-M04 | 83.06 |
| 10 | F20-M04 | 81.89 | F18-M10 | 82.27 | F05-M09 | 80.33 | F03-M06 | 82.78 |

[Table 9]

| Child Individuals | O-05 | | O-06 | | O-07 | | O-07 | |
|---|---|---|---|---|---|---|---|---|
| Number of Reads | 357 | | 356 | | 354 | | 351 | |
| Rank | Parent Candidate | Matching Accuracy (%) | Parent Candidate | Matching Accuracy (%) | Parent Candidate | Matching Accuracy (%) | Parent Candidate | Matching Accuracy (%) |
| 1 | F17-M02 | 100.00 | F05-M09 | 100.00 | F05-M11 | 100.00 | F05-M11 | 100.00 |
| 2 | F08-M02 | 84.87 | F08-M09 | 87.92 | F08-M11 | 85.88 | F05-M03 | 83.19 |
| 3 | F04-M02 | 82.63 | F16-M09 | 86.80 | F16-M11 | 83.05 | F08-M11 | 82.91 |
| 4 | F17-M03 | 82.63 | F05-M05 | 84.27 | F15-M11 | 81.07 | F05-M05 | 81.77 |
| 5 | F02-M02 | 81.79 | F06-M09 | 83.71 | F04-M11 | 80.79 | F05-M02 | 81.20 |
| 6 | F09-M02 | 80.67 | F03-M09 | 83.43 | F13-M11 | 79.94 | F05-M08 | 81.20 |
| 7 | F 14-M02 | 80.67 | F12-M09 | 83.15 | F12-M11 | 79.38 | F05-M07 | 80.91 |
| 8 | F06-M02 | 80.11 | F17-M09 | 83.15 | F03-M11 | 79.10 | F16-M11 | 80.91 |
| 9 | F17-M09 | 80.11 | F18-M09 | 82.87 | F17-M11 | 78.53 | F05-M10 | 80.63 |
| 10 | F16-M02 | 79.83 | F04-M09 | 82.58 | F06-M11 | 78.25 | F05-M09 | 80.06 |

[Table 10]

| Child Individuals | O-09 | | O-10 | |
|---|---|---|---|---|
| Number of Reads | 348 | | 244 | |
| Rank | Parent Candidate | Matching Accuracy (%) | Parent Candidate | Matching Accuracy (%) |
| 1 | F05-M02 | 100.00 | F17-M11 | 100.00 |
| 2 | F08-M02 | 88.22 | F17-M03 | 86.07 |
| 3 | F16-M02 | 86.21 | F17-M07 | 86.07 |
| 4 | F06-M02 | 83.62 | F17-M04 | 84.02 |
| 5 | F04-M02 | 82.76 | F17-M06 | 83.20 |
| 6 | F12-M02 | 82.47 | F04-M11 | 82.79 |
| 7 | F13-M02 | 82.47 | F17-M08 | 82.79 |
| 8 | F05-M03 | 81.61 | F17-M02 | 82.38 |
| 9 | F10-M02 | 81.61 | F17-M05 | 82.38 |
| 10 | F01-M02 | 81.32 | F08-M11 | 81.97 |

As shown in Tables 8 to 10, one set of parent candidates having a matching accuracy of 100% exists for each child individual. It is estimated that the combination of the parent candidates is a parent combination of child individuals.

Although the embodiment of the invention has been described in detail with reference to the drawings, the specific configuration is not limited to the embodiment, and a design or the like within a scope not departing from the gist of the invention is also included.

Reference Sign List

    100 genetic information analysis system
    1 reference sequence information acquisition device
    2 evaluation device
    3 specific sequence information acquisition device
    11 control unit
    12 input unit
    13 communication unit
    14 storage unit
    15 output unit
    21 control unit
    22 input unit
    23 communication unit
    24 storage unit
    25 output unit
    31 control unit
    32 input unit
    33 communication unit
    34 storage unit
    35 output unit
    111 reference-side information acquisition unit
    112 reference sequence information acquisition unit
    113 recording unit
    114 output control unit
    211 comparison-side information acquisition unit
    212 accuracy acquisition unit

213 recording unit
214 output control unit
311 other-variety-side information acquisition unit
312 specific sequence information acquisition unit
313 recording unit
314 output control unit
91 processor
92 memory
93 processor
94 memory
95 processor
96 memory

**Claims**

1. A genetic information analysis system, comprising:

   a comparison-side information acquisition unit configured to acquire comparison target sequence information indicating a comparison target sequence which is a base sequence of genetic information on a comparison target; and
   an accuracy acquisition unit configured to acquire an accuracy that the genetic information on the comparison target is the same as genetic information on an analysis target, based on the comparison target sequence information and reference sequence information indicating a reference sequence selected from base sequences common to two or more independently acquired base sequences of the genetic information on the analysis target.

2. The genetic information analysis system according to claim 1, wherein
   the reference sequence information is acquired based on at least first base sequence information indicating one of the two or more independently acquired base sequences of the genetic information on the analysis target, and second base sequence information indicating another base sequence of the two or more base sequences.

3. The genetic information analysis system according to claim 1 or 2, wherein
   a sequence information amount of the base sequence of the comparison target is larger than a sequence information amount of the reference sequence.

4. A genetic information analysis method, comprising:

   a step of acquiring comparison target sequence information indicating a comparison target sequence which is a base sequence of genetic information on a comparison target; and
   a step of acquiring an accuracy that the genetic information on the comparison target is the same as genetic information on an analysis target, based on the comparison target sequence information and reference sequence information indicating a reference sequence selected from base sequences common to two or more independently acquired base sequences of the genetic information on the analysis target.

5. The genetic information analysis method according to claim 4, wherein
   the reference sequence information is acquired based on at least first base sequence information indicating one of the two or more independently acquired base sequences of the genetic information on the analysis target, and second base sequence information indicating another base sequence of the two or more base sequences.

6. The genetic information analysis method according to claim 4 or 5, wherein
   a sequence information amount of the base sequence of the comparison target is larger than a sequence information amount of the reference sequence.

7. A reference sequence information acquisition device, comprising:
   a reference sequence information acquisition unit configured to acquire reference sequence information indicating a reference sequence selected from base sequences common to two or more independently acquired base sequences of genetic information on an analysis target.

8.  A reference sequence information acquisition method, comprising:
    a step of acquiring reference sequence information indicating a reference sequence selected from base sequences common to two or more independently acquired base sequences of genetic information on an analysis target.

9.  A specific sequence information acquisition system, comprising:

    a reference sequence information acquisition unit configured to acquire reference sequence information indicating a reference sequence selected from base sequences common to two or more independently acquired base sequences of genetic information on an analysis target; and
    a specific sequence information acquisition unit configured to acquire, from the reference sequence information, specific sequence information indicating a base sequence that is specifically present in the reference sequence.

10. A specific sequence information acquisition method, comprising:

    a step of acquiring reference sequence information indicating a reference sequence selected from base sequences common to two or more independently acquired base sequences of genetic information on an analysis target; and
    a step of acquiring, from the reference sequence information, specific sequence information indicating a base sequence that is specifically present in the reference sequence.

11. A method for producing a detection nucleic acid, comprising:

    a step of acquiring specific sequence information by the method according to claim 10; and
    a step of producing a detection nucleic acid of the analysis target based on the specific sequence information.

[FIG. 1]

FIRST BASE SEQUENCE
INFORMATION

SECOND BASE SEQUENCE
INFORMATION

COMPARISON TARGET
SEQUENCE INFORMATION

GENETIC INFORMATION
ANALYSIS SYSTEM

100

1

REFERENCE SEQUENCE
INFORMATION
ACQUISITION DEVICE

2

EVALUATION DEVICE

REFERENCE SEQUENCE
INFORMATION

[FIG. 2]

FIRST BASE SEQUENCE

BASE SEQUENCE (1-1) ————

BASE SEQUENCE (1-2) ————

BASE SEQUENCE (1-m) ————

SECOND BASE SEQUENCE

BASE SEQUENCE (2-1) ————

BASE SEQUENCE (2-2) ————

BASE SEQUENCE (2-n) ————

REFERENCE SEQUENCE

BASE SEQUENCE (1-i1) ———— BASE SEQUENCE (2-j1) ———— MATCH

BASE SEQUENCE (1-i2) ———— BASE SEQUENCE (2-j2) ———— MATCH

BASE SEQUENCE (1-ix) ———— BASE SEQUENCE (2-jx) ———— MATCH

[FIG. 3]

REFERENCE SEQUENCE INFORMATION ACQUISITION DEVICE 1

CONTROL UNIT 11

PROCESSOR 91

MEMORY 92

STORAGE UNIT 14

INPUT UNIT 12

OUTPUT UNIT 15

COMMUNICATION UNIT 13

[FIG. 4]

2

EVALUATION DEVICE

21

CONTROL UNIT

93

PROCESSOR

94

MEMORY

24

STORAGE UNIT

22

INPUT UNIT

25

OUTPUT

23

COMMUNICATION UNIT

[FIG. 5]

11

CONTROL UNIT

111

| REFERENCE-SIDE INFORMATION ACQUISITION UNIT |

112

| REFERENCE SEQUENCE INFORMATION ACQUISITION UNIT |

113

| RECORDING UNIT |

114

| OUTPUT CONTROL UNIT |

[FIG. 6]

21

CONTROL UNIT

211

COMPARISON-SIDE INFORMATION
ACQUISITION UNIT

212

ACCURACY ACQUISITION UNIT

213

RECORDING UNIT

214

OUTPUT CONTROL UNIT

[FIG. 7]

START

S101

ACQUIRE FIRST BASE SEQUENCE INFORMATION AND
SECOND BASE SEQUENCE INFORMATION

S102

RECORD REFERENCE SEQUENCE INFORMATION

S103

RECORD REFERENCE SEQUENCE INFORMATION

END

[FIG. 8]

```
          ┌─────────────┐
          │    START    │
          └─────────────┘
                 │
                 ▼                                    S201
┌──────────────────────────────────────────────────┐
│   ACQUIRE REFERENCE SEQUENCE INFORMATION AND       │
│    COMPARISON TARGET SEQUENCE INFORMATION          │
└──────────────────────────────────────────────────┘
                 │
                 ▼                                    S202
┌──────────────────────────────────────────────────┐
│           ACQUIRE MATCHING ACCURACY                │
└──────────────────────────────────────────────────┘
                 │
                 ▼                                    S203
┌──────────────────────────────────────────────────┐
│           DISPLAY MATCHING ACCURACY                │
└──────────────────────────────────────────────────┘
                 │
                 ▼
          ┌─────────────┐
          │     END     │
          └─────────────┘
```

[FIG. 9]

SEGMENT

[FIG. 10]

**SPECIFIC SEQUENCE INFORMATION ACQUISITION DEVICE** (2)

**CONTROL UNIT** (31)
- **PROCESSOR** (95)
- **MEMORY** (96)

**STORAGE UNIT** (34)

**INPUT UNIT** (32)

**OUTPUT UNIT** (35)

**COMMUNICATION UNIT** (33)

[FIG. 11]

31

CONTROL UNIT

311

OTHER-VARIETY-SIDE
INFORMATION ACQUISITION UNIT

312

SPECIFIC SEQUENCE
INFORMATION ACQUISITION UNIT

313

RECORDING UNIT

314

OUTPUT CONTROL UNIT

[FIG. 12]

START

S301

ACQUIRE REFERENCE SEQUENCE INFORMATION AND
OTHER-VARIETY SEQUENCE INFORMATION

S302

ACQUIRE SPECIFIC SEQUENCE INFORMATION

S303

DISPLAY SPECIFIC SEQUENCE INFORMATION

END

[FIG. 13]

[FIG. 14]

```
┌─────────────┐       ┌─────────────┐       ┌─────────────┐
│  STRAIN (a) │───────│  STRAIN (b) │───────│  STRAIN (c) │
└─────────────┘   │   └─────────────┘   │   └─────────────┘
                  │                     │
            ┌─────────────┐       ┌─────────────┐       ┌─────────────┐
            │  VARIETY A  │       │  VARIETY B  │───────│  STRAIN (d) │
            └─────────────┘       └─────────────┘   │   └─────────────┘
                                                    │
                                              ┌─────────────┐
                                              │  VARIETY C  │
                                              └─────────────┘


┌─────────────┐                       ┌─────────────┐              ┌─────────────┐
│  VARIETY D  │───────────┬───────────│  VARIETY E  │              │  VARIETY H  │
└─────────────┘           │           └─────────────┘              └─────────────┘
      │                   │
SO-CALLED                 │
 INBRED                   │
┌─────────────┐     ┌─────────────┐
│  VARIETY F  │     │  VARIETY G  │
└─────────────┘     └─────────────┘
```

[FIG. 15]

[FIG. 16]

[FIG. 17]

[FIG. 18]

**INTERNATIONAL SEARCH REPORT**

| International application No. |
| --- |
| PCT/JP2021/003874 |

**A. CLASSIFICATION OF SUBJECT MATTER**
C12Q 1/6869(2018.01)i
FI: C12Q1/6869 Z

According to International Patent Classification (IPC) or to both national classification and IPC

**B. FIELDS SEARCHED**

Minimum documentation searched (classification system followed by classification symbols)
C12Q1/6869

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BISIS/WPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2018-513508 A (PERSONAL GENOME DIAGNOSTICS INC.) 24 May 2018 (2018-05-24) claims, paragraphs [0004], [0082]-[0138] | 1-11 |
| X | WO 2019/169042 A1 (CORNELL UNIVERSITY) 06 September 2019 (2019-09-06) claims, paragraphs [0142]-[0173] | 1-11 |
| X | JP 2017-510244 A (ACCURAGEN INC.) 13 April 2017 (2017-04-13) claims, paragraphs [0060], [0061] | 1-11 |

☒ Further documents are listed in the continuation of Box C.   ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 29 March 2021 (29.03.2021) | 20 April 2021 (20.04.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2021/003874 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | 陶山佳久，森林遺伝育種学的研究における MIG-seq 法の利用，森林遺伝育種 第 8 巻 (2019), pp. 85-89, in particular, principle of MIG-seq, analysis of inter-individual levels (clone identification·cariety identification), fig. -1, non-official translation (SUYAMA, Yoshihisa. Use of MIG-seq method for Research in forest genetics and tree breeding. Forest genetics and tree breeding vol. 8 (2019).) | 1-11 |
| A | SUYAMA, Y. et al., MIG-seq:an effective PCR-based method for genome-wide single-nucleotide polymorphism genotyping using the next-generation sequencing platform, Scientific Reports, (2015), vol. 5, no. 16963, pp. 1-12, abstract, fig. 1, methods | 1-11 |

Form PCT/ISA/210 (continuation of second sheet) (January 2015)

**INTERNATIONAL SEARCH REPORT**
Information on patent family members

International application No.

PCT/JP2021/003874

| Patent Documents referred in the Report | Publication Date | Patent Family | Publication Date |
|---|---|---|---|
| JP 2018-513508 A | 24 May 2018 | US 2016/0273049 A1 claims, paragraphs [0004], [0082]-[0138] EP 3271848 A1 | |
| WO 2019/169042 A1 | 06 Sep. 2019 | (Family: none) | |
| JP 2017-510244 A | 13 Apr. 2017 | US 2016/0304954 A1 claims, paragraphs [0060], [0061] EP 3080298 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6220332 B

**Non-patent literature cited in the description**

- **SUYAMA Y ; MATSUKI Y.** MIG-seq: an effective PCR-based method for genome-wide single-nucleotide polymorphism genotyping using the next-generation sequencing platform. *Scientific Reports,* 2015, vol. 5, 16963
- **TRAVIS C GLENN.** *Mol Ecol Resour,* September 2011, vol. 11 (5), 759-69
- **YOSHIHISA SUYAMA.** Use of MIG-seq in forest genetic and breeding studies. *Forest Genetics and Breeding,* 2019, vol. 8 (2), 85-89
- *Nat. Bioltechnol.,* 2008, vol. 26, 1146-1153
- *SCIENCE,* 2004, vol. 303, 1189-1192
- *Surface Science,* 1999, vol. 432, L611-L616
- *Nano Lett,* 2011, 279-285
- *Scientific Reports,* 2012, vol. 2, 501
- **SUYAMA Y ; MATSUKI Y.** Multiplexed ISSR Genotyping. *Scientific Reports,* 2015, vol. 5, 16963
- **SUYAMA Y ; MATSUKI Y.** Multiplexed ISSR Genotyping by sequencing. *Scientific Reports,* 2015, vol. 5, 16963
- **YOSHIHISA SUYAMA.** Use of MIG-seq in forest genetic and breeding studies. *Forest Genetics and Breeding,* 2019, vol. 8 (2), 85-89